(19) Europäisches Patentamt
European Patent Office
Office européen des brevets

(11) EP 2 756 067 B1

(12) # EUROPEAN PATENT SPECIFICATION

(45) Date of publication and mention of the grant of the patent:
**14.06.2017 Bulletin 2017/24**

(21) Application number: **12766771.5**

(22) Date of filing: **12.09.2012**

(51) Int Cl.:
***C11D 3/50*** *(2006.01)* ***A01N 25/28*** *(2006.01)*
***B01J 13/00*** *(2006.01)*

(86) International application number:
**PCT/US2012/054747**

(87) International publication number:
**WO 2013/039963 (21.03.2013 Gazette 2013/12)**

(54) **MICROCAPSULE COMPOSITIONS COMPRISING PH TUNEABLE DI-AMIDO GELLANTS**

MIKROKAPSELZUSAMMENSETZUNGEN MIT PH-EINSTELLBAREN DI-AMIDOGELIERMITTELN

COMPOSITIONS DE MICROCAPSULE CONTENANT DES GÉLIFIANTS DI-AMIDO SENSIBLES AU PH

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.09.2011 US 201161533816 P**

(43) Date of publication of application:
**23.07.2014 Bulletin 2014/30**

(73) Proprietor: **The Procter & Gamble Company**
**Cincinnati, OH 45202 (US)**

(72) Inventors:
- **FERNANDEZ PRIETO, Susana**
**1853 Strombeek-Bever (BE)**
- **SMETS, Johan**
**B-3210 Lubbeek (BE)**

(74) Representative: **Siddiquee, Sanaul Kabir**
**N.V. Procter & Gamble**
**Services Company S.A.**
**Temselaan 100**
**1853 Strombeek-Bever (BE)**

(56) References cited:
**EP-A1- 2 365 052** **US-A1- 2006 040 844**
**US-A1- 2010 150 975**

- **ESTROFF L A ET AL: "WATER GELATION BY SMALL ORGANIC MOLECULES", CHEMICAL REVIEWS, AMERICAN CHEMICAL SOCIETY, US, vol. 104, no. 3, 1 January 2004 (2004-01-01), pages 1201-1218, XP003016413, ISSN: 0009-2665, DOI: 10.1021/CR0302049**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 756 067 B1

## Description

TECHNICAL FIELD

Embodiments disclosed herein are directed, in general, to microcapsule compositions. That is, compositions comprising microcapsules which are generally suspended in the composition. In particular, embodiments are directed to microcapsule compositions comprising pH-tuneable di-amido gellants, to detergent compositions comprising the microcapsule compositions, and to processes for making the compositions.

BACKGROUND

Many kinds of encapsulates are known as vehicles for carrying one or more benefit agents in various consumer products such as, for example, fluid laundry detergents. For example, benefit agents such as perfumes, drugs, and biocontrol agents such as antibacterials may be incorporated into compositions by encapsulating the benefit agents at the core of microcapsules having polymeric shell walls.

Microcapsules are by nature fragile and are typically manufactured in aqueous slurries. The aqueous slurries may be temporarily stored apart from the manufacturing site and/or transported from their points of manufacture to other locations, at which they are mixed into final products. The mixing of the slurries during transportation or exposure to high temperatures during storage often causes a very small percentage of microcapsules to rupture or otherwise leach its benefit agent into the slurry composition. Traditional slurry compositions may be sensitive to this inadvertent addition of benefit agent. Traditional slurries comprising, for example, polysaccharide derivative structurants loose their viscosities and become unstable, thereby becoming ineffective in delivering a homogeneous slurry composition. Especially for long supply chains or long storage conditions and/or at elevated temperatures, the PMC slurries with traditional polysaccharide derived structurants show a tendency for phase split or physical instability induced by perfume compatibility of the structurant, agglomeration of the particles, and density differences between the particles and the slurry.

As such, a need remains for microcapsule compositions that have acceptable rheologies and rheological stability over time, even during transport, especially at elevated temperatures.

SUMMARY

The foregoing needs are met, at least in part, through the embodiments of microcapsule compositions disclosed herein, which include at least one pH-tuneable di-amido gellant as a structuring agent.

In some embodiments, a microcapsule composition may include a vehicle such as water, a population of encapsulates comprising a core and a shell wall surrounding the core. The core comprises at least one benefit agent, and the shell wall comprises at least one polymer. The microcapsule composition further includes a pH-tuneable di-amido gellant.

In further embodiments, a consumer product, in particular embodiments a fluid laundry detergent composition, includes a microcapsule composition and at least one adjunct ingredient. The microcapsule composition may include a vehicle such as water, a population of encapsulates comprising a core and a shell wall at least partially surrounding the core. The core contains at least one benefit agent, and the shell wall includes at least one polymer. The microcapsule composition further includes a pH-tuneable amido gellant. The at least one adjunct ingredient may be selected from surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments, for example.

DETAILED DESCRIPTION

Embodiments disclosed herein are directed to microcapsule compositions and to fluid detergent compositions including the microcapsule compositions.

<u>Microcapsule Compositions</u>

The compositions of the present invention may comprise a microcapsule composition such as, for example, a perfume microcapsule composition. The microcapsule composition is preferably in the form of a slurry or suspension including a vehicle, a population of encapsulates, a pH-tuneable di-amido gellant and, optionally, a parametric balancing agent. The encapsulates in the microcapsule composition may comprise a core material and a shell wall material that at least partially surrounds the core. The core material may comprise a benefit agent, as detailed below, of which a

perfume is one non-limiting embodiment. The encapsulate shell wall material, which alternatively may be described as an encapsulate shell material, is a polymeric structure. A microcapsule composition in the form of a slurry may comprise water as the vehicle and, as such, may be described as an aqueous slurry. US 2010/0150975 describes structured liquid compositions comprising microcapsules. US 2006/0040844 discloses liquid detergent compositions comprising microcapsules. Water gelation by small molecules such as diamido gellants has been described in Chemical Reviews, Vol. 104, No. 3 (2004), pages 1201-1217. In one embodiment, at least about 75%, about 85% or even about 90% of the encapsulates may have a particle size of from about 1 μm to about 1500 μm, about 5 μm to 500 μm, from about 10 μm to about 200 μm, or even from about 15 μm to about 80 μm. The shell wall of the encapsulate may have a thickness of from about 60 nm to about 250 nm, from about 80 nm to about 180 nm, or even from about 100 nm to about 160 nm.

*Vehicle*

In one aspect of said microcapsule composition, said vehicle may comprise water, vegetal oils, and/or organic solvents. In one aspect, the vehicle may be water. In other aspects, the vehicle may further comprise additives such as salts, perfumes, biocides, polymers and mixtures thereof.

*pH Tuneable di-Amido Gellant*

The microcapsule composition preferably comprises at least one pH-tuneable di-amido gellant. An microcapsule composition may comprise, for example, from about 0.01% to about 5%, or from about 0.1% to about 2%, by weight of the microcapsule composition, of the pH tuneable amido gellant.

The pH tuneable di-amido gellant provides the microcapsule composition with a viscosity profile that is dependent on the pH of the composition. The pH tuneable amido gellants comprise at least one pH sensitive group. When a pH tuneable di-amido gellant is added to a polar protic solvent such as water, it is believed that the nonionic species form the viscosity building network while the ionic species are soluble and do not form a viscosity building network. By increasing or decreasing the pH (depending on the selection of the pH-sensitive groups) the di-amido gellant is either protonated or deprotonated. Thus, by changing the pH of the solution, the solubility, and hence the viscosity building behavior, of the di-amido gellant can be controlled. By careful selection of the pH-sensitive groups, the $pK_a$ of the di-amido gellant can be tailored. Hence, the choice of the pH-sensitive groups can be used to select the pH at which the di-amido gellant builds viscosity.

The pH tuneable di-amido gellant has the formula:

[I] 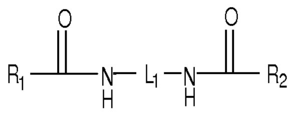

wherein $R_1$ and $R_2$ are aminofunctional end-groups; $L_1$ is a backbone moiety having molecular weight from 14 g/mol to 500 g/mol; and at least one of $L_1$, $R_1$ and $R_2$ comprises a pH-sensitive group and;
wherein the pH tuneable di-amido gellant has a $pK_a$ of from 1 to 30, preferably a $pK_a$ of from 1.5 to 14.

The pH tuneable di-amido gellant comprises at least one amido functional group, and further comprises at least one pH-sensitive group. Preferably, the pH tuneable di-amido gellant has a molecular weight from 150 to 1500 g/mol, more preferably from 300 g/mol to 900 g/mol, most preferably from 400 g/mol to 700 g/mol.

$L_1$ preferably has the formula:

[III] $$L_1 = A_a\text{-}B_b\text{-}C_c\text{-}D_d,$$

wherein: (a+b+c+d) is from 1 to 20; and A, B, C and D are independently selected from the linking groups consisting of:

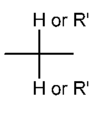 , 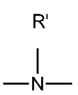 , 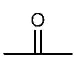 , 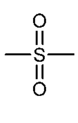 , 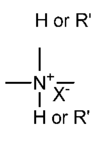 , 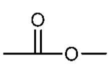 , 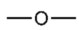 , 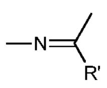 , 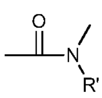 , 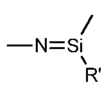 ,

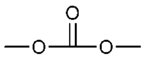 , 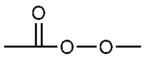 , 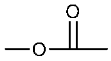 , 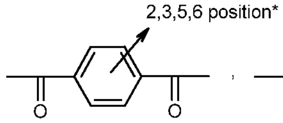 , 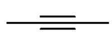 , 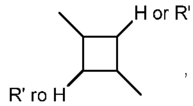 ,

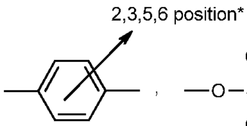 , 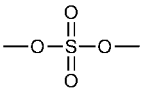 , 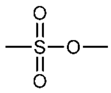 , 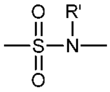 , 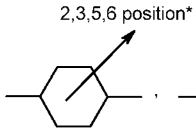 , 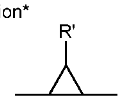 , 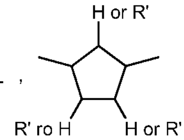 ,

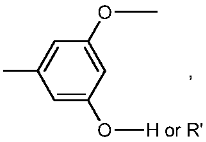 , 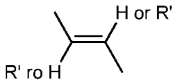

Preferably, A, B, C and D are independently selected from the linking groups consisting of:

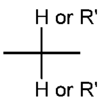 , 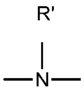 , 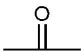 , 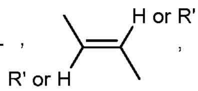 , 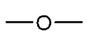 , 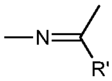 , 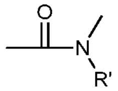 , 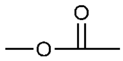 ,

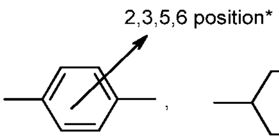 , 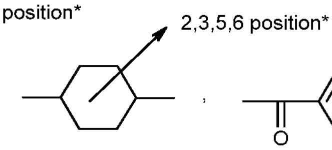 , 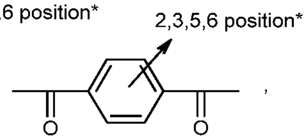 , 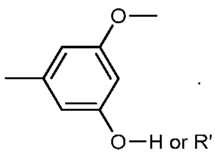 .

*the arrow indicates up to 4 substitutions in the positions indicated, and X- an anion

Preferably, $L_1$ is selected from $C_2$ to $C_{20}$ hydrocarbyl chains, more preferably $C_6$ to $C_{12}$, most preferably $C_8$ to $C_{10}$.

In a preferred embodiment: $R_1$ is $R_3$ or

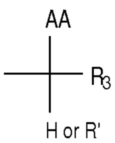

,

$R_2$ is $R_4$ or

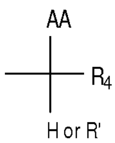

,

wherein each AA is independently selected from the group consisting of:

R'' , H , $-CH_3$ , 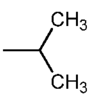 , 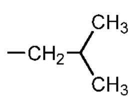 , $-CH_2-OH$ , 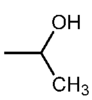 , $-CH_2-SH$ , $-CH_2-CH_2-S-CH_3$ ,

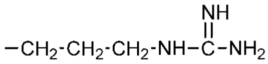 , 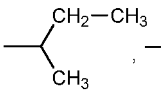 , $-CH_2-CH_2-CH_2-CH_2-NH_2$ , 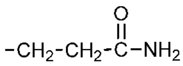 , 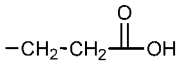 ,

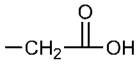 , 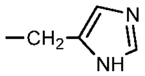 , 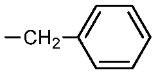 , 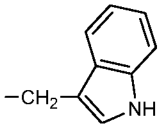 , 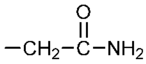 , 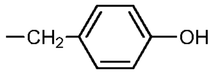

and $R_3$ and $R_4$ independently have the formula

[IV] $(L')_o-(L'')_q-R$,

wherein: (o + q) is from 1 to 10; L' and L" are linking groups, independently selected from the same groups as A, B, C and D in equation [III]; and R, R' and R" are independently selected either from the pH sensitive groups listed under AA or the pH-sensitive-groups consisting of:

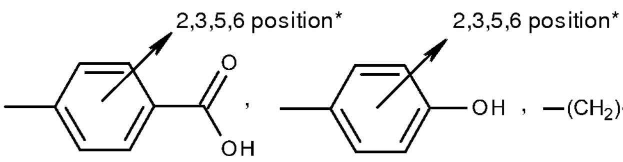

, 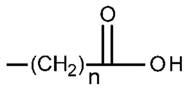 , 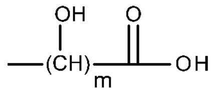 , 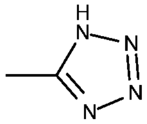 ,

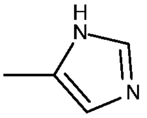 , 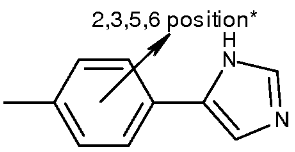

, 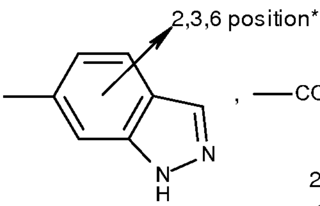

, —COOH , 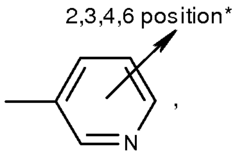

,

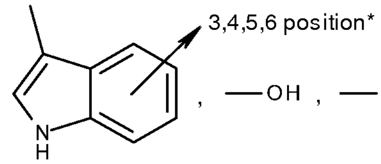

, —OH , —SH , —$NH_2$ , —CN , 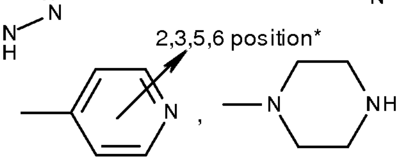

,

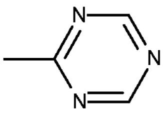 .

*the arrow indicates up to 4 substitutions in the positions indicated, n and m are integers from 1 to 20 or from the non-pH-sensitive groups listed under AA and/or from the groups consisting of: -H -$CH_3$, -$COOCH_3$,

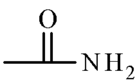 , 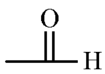 , 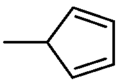 , 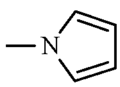 ,

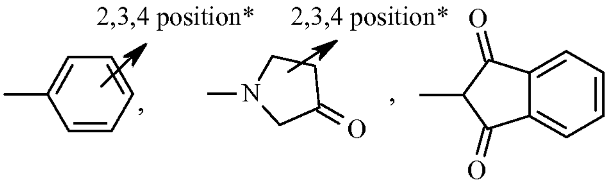

, 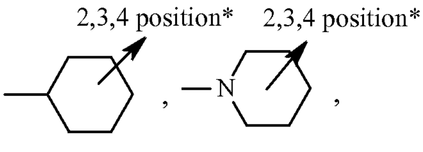

,

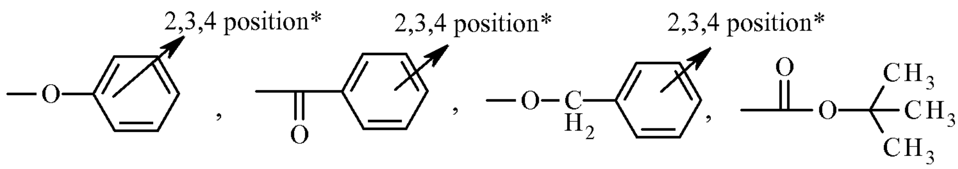

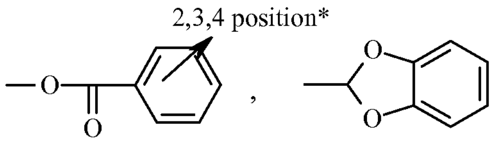

such that at least one of R, R' and R" comprises a pH-sensitive group. Preferably, R comprises the pH-sensitive group.

In other embodiments, at least some of R, R' and R" are independently selected from the group of pH-sensitive molecules consisting of:

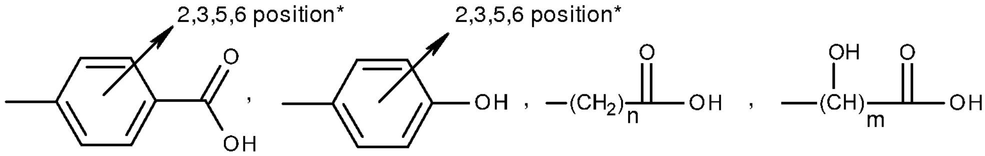

-COOH,

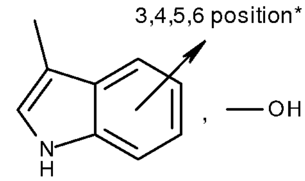 , —OH , —$NH_2$ , 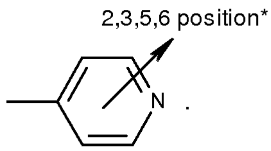 .

In a preferred embodiment, the pH tuneable di-amido gellant having structure [I] is characterized in that: $L_1$ is an aliphatic linking group with a backbone chain of from 2 to 20 carbon atoms, preferably -$(CH_2)_n$- wherein n is selected from 2 to 20, and both $R_1$ and $R_2$ have the structure:

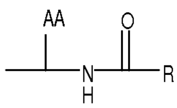

wherein AA is preferably selected from the group consisting of:

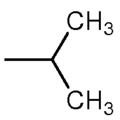 , 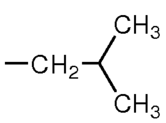 , 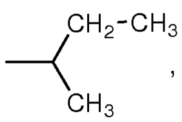 , $-CH_2-CH_2-S-CH_3$ , 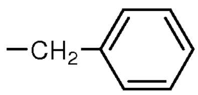 ,

and R is preferably selected from the pH-sensitive groups consisting of: -OH,

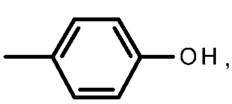 , 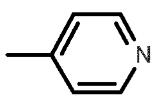 , —COOH , 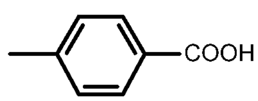 .

In another embodiment, two or more of $L_1$, L' and L" are the same group.

The pH tuneable di-amido gellant molecule described in formula [I] can be symmetric with respect to the $L_1$ entity or can be asymmetric. Without intending to be bound by theory, it is believed that symmetric pH tuneable di-amido

gellant molecules allow for more orderly structured networks to form, whereas compositions comprising one or more asymmetric pH tuneable di-amido gellant molecules can create disordered networks.

Suitable pH tuneable amido gellants having structure [I] may be selected from table 1 and mixtures thereof.

*Illustrative embodiments of pH tuneable di-amido gellants:*

TABLE 1: Non-limiting examples of pH tuneable di-amido gellants having structure [I]

| | |
|---|---|
| 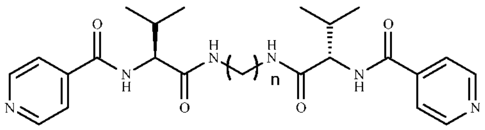 | |
| *N*,*N*’-(2*S*,2’*S*)-1,1’-(ethane-1,2-diylbis(azanediyl))bis(3-methyl-1-oxobutane-2,1-diyl)diisonicotinamide | *N*,*N*’-(2*S*,2’*S*)-1,1’-(propane-1,3-diylbis(azanediyl))bis(3-methyl-1-oxobutane-2,1-diyl)diisonicotinamide |
| *N*,*N*’-(2*S*,2’*S*)-1,1’-(butane-1,4-diylbis(azanediyl))bis(3-methyl-1-oxobutane-2,1-diyl)diisonicotinamide | *N*,*N*’-(2*S*,2’*S*)-1,1’-(pentane-1,5-diylbis(azanediyl))bis(3-methyl-1-oxobutane-2,1-diyl)diisonicotinamide |
| *N*,*N*’-(2*S*,2’*S*)-1,1’-(hexane-1,6-diylbis(azanediyl))bis(3-methyl-1-oxobutane-2,1-diyl)diisonicotinamide | *N*,*N*’-(2*S*,2’*S*)-1,1’-(heptane-1,7-diylbis(azanediyl))bis(3-methyl-1-oxobutane-2,1-diyl)diisonicotinamide |
| *N*,*N*’-(2*S*,2’*S*)-1,1’-(octane-1,8-diylbis(azanediyl))bis(3-methyl-1-oxobutane-2,1-diyl)diisonicotinamide | *N*,*N*’-(2*S*,2’*S*)-1,1’-(nonane-1,9-diylbis(azanediyl))bis(3-methyl-1-oxobutane-2,1-diyl)diisonicotinamide |
| *N*,*N*’-(2*S*,2’*S*)-1,1’-(decane-1,10-diylbis(azanediyl))bis(3-methyl-1-oxobutane-2,1-diyl)diisonicotinamide | *N*,*N*’-(2S,2’*S*)-1,1’-(undecane-1,11-diylbis(azanediyl))bis(3-methyl-1-oxobutane-2,1-diyl)diisonicotinamide |
| *N*,*N*’-(2*S*,2’*S*)-1,1’-(dodecane-1,12-diylbis(azanediyl))bis(3-methyl-1-oxobutane-2,1-diyl)diisonicotinamide | *N*,*N*’-(2*S*,2’*S*)-1,1’-(tridecane-1,13-diylbis(azanediyl))bis(3-methyl-1-oxobutane-2,1-diyl)diisonicotinamide |
| *N*,*N*’-(2*S*,2’*S*)-1,1’-(tetradecane-1,14-diylbis(azanediyl))bis(3-methyl-1-oxobutane-2,1-diyl)diisonicotinamide | *N*,*N*’-(2*S*,2’*S*)-1,1’-(hexadecane-1,16-diylbis(azanediyl))bis(3-methyl-1-oxobutane-2,1-diyl)diisonicotinamide |
| *N*,*N*’-(2*S*,2’*S*)-1,1’-(octadecane-1,18-diylbis(azanediyl))bis(3-methyl-1-oxobutane-2,1-diyl)diisonicotinamide | |
| 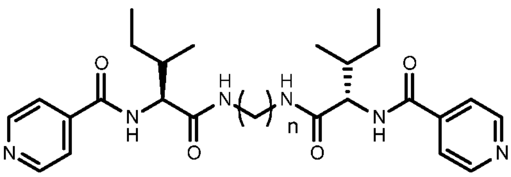 | |
| *N*-[(1*S*)-2-methyl-1-[2-[[(2*S*)-3-methyl-2-(pyridine-4-carbonylamino)pentanoyl]amino]ethylcarbamoyl]butyl]pyridine-4-carboxamide | *N*-[(1*S*)-2-methyl-1-[3-[[(2*S*)-3-methyl-2-(pyridine-4-carbonylamino)pentanoyl]amino]propylcarbamoyl]butyl]pyridine-4-carboxamide |
| *N*-[(1*S*)-2-methyl-1-[4-[[(2*S*)-3-methyl-2-(pyridine-4-carbonylamino)pentanoyl]amino]butylcarbamoyl]butyl]pyridine-4-carboxamide | *N*-[(1*S*)-2-methyl-1-[5-[[(2*S*)-3-methyl-2-(pyridine-4-carbonylamino)pentanoyl]amino]pentylcarbamoyl]butyl]pyridine-4-carboxamide |

(continued)

| | |
|---|---|
| *N*-[(1*S*)-2-methyl-1-[6-[[(2*S*)-3-methyl-2-(pyridine-4-carbonylamino)pentanoyl]amino]hexylcarbamoyl]butyl]pyridine-4-carboxamide | *N*-[(1*S*)-2-methyl-1-[7-[[(2*S*)-3-methyl-2-(pyridine-4-carbonylamino)pentanoyl]amino]heptylcarbamoyl]butyl]pyridine-4-carboxamide |
| *N*-[(1*S*)-2-methyl-1-[8-[[(2*S*)-3-methyl-2-(pyridine-4-carbonylamino)pentanoyl]amino]octylcarbamoyl]butyl]pyridine-4-carboxamide | *N*-[(1*S*)-2-methyl-1-[9-[[(2*S*)-3-methyl-2-(pyridine-4-carbonylamino)pentanoyl]amino]nonylcarbamoyl]butyl]pyridine-4-carboxamide |
| *N*-[(1*S*)-2-methyl-1-[10-[[(2*S*)-3-methyl-2-(pyridine-4-carbonylamino)pentanoyl]amino]decylcarbamoyl]butyl]pyridine-4-carboxamide | *N*-[(1*S*)-2-methyl-1-[11-[[(2*S*)-3-methyl-2-(pyridine-4-carbonylamino)pentanoyl]amino]undecylcarbamoyl]butyl]pyridine-4-carboxamide |
| *N*-[(1*S*)-2-methyl-1-[12-[[(2*S*)-3-methyl-2-(pyridine-4-carbonylamino)pentanoyl]amino]dodecycarbamoyl]butyl]pyridine-4-carboxamide | |
| 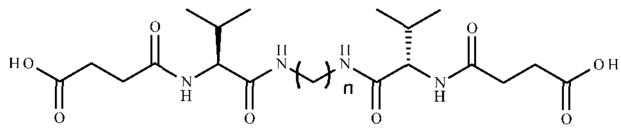 | |
| | *(6S,* 13*S*)-6,13-diisopropyl-4,7,12,15-tetraoxo-5,8,11,14-tetraazaoctadecane-1,18-dioic acid |
| (6*S*,14*S'*)-6,14-diisopropyl-4,7,13,16-tetraoxo-5,8,12,15-tetraazanonadecane-1,19-dioic acid | (6*S*,15*S*)-6,15-diisopropyl-4,7,14,17-tetraoxo-5,8,13,16-tetraazaeicosane-1,20-dioic acid |
| (6*S*,16*S*)-6,16-diisopropyl-4,7,15,18-tetraoxo-5,8,14,17-tetraazaheneicosane-1,21-dioic acid | (6*S*,17*S*)-6,17-diisopropyl-4,7,16,19-tetraoxo-5,8,15,18-tetraazadocosane-1,22-dioic acid |
| (6*S*,18*S*)-6,18-diisopropyl-4,7,17,20-tetraoxo-5,8,16,19-tetraazatricosane-1,23-dioic acid | (6*S*,19*S*)-6,19-diisopropyl-4,7,18,21-tetraoxo-5,8,17,20-tetraazatetracosane-1,24-dioic acid |
| (6*S*,20*S*)-6,20-diisopropyl-4,7,19,22-tetraoxo-5,8,18,21-tetraazapentacosane-1,25-dioic acid | (6*S*,21*S*)-6,21-diisopropyl-4,7,20,23-tetraoxo-5,8,19,22-tetraazahexacosane-1,26-dioic acid |
| (6*S*,22*S*)-6,22-diisopropyl-4,7,21,24-tetraoxo-5,8,20,23-tetraazaheptacosane-1,27-dioic acid | (6*S*,23*S*)-6,23-diisopropyl-4,7,22,25-tetraoxo-5,8,21,24-tetraazaoctacosane-1,28-dioic acid |
| 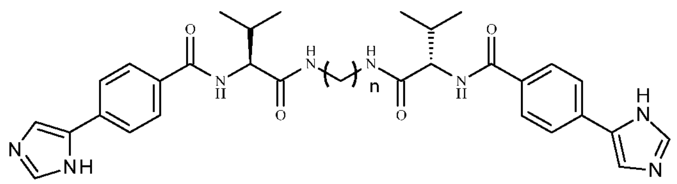 | |
| *N*,*N'*-(2*S*,2'*S*)-1,1'-(ethane-1,2-diylbis(azanediyl))bis(3-methyl-1-oxobutane-2,1-diyl)bis(4-(1*H*-imidazol-5-yl)benzamide) | *N*,*N'*-(2*S*,2'*S*)-1,1'-(propane-1,3-diylbis(azanediyl))bis(3-methyl-1-oxobutane-2,1-diyl)bis(4-(1*H*-imidazol-5-yl)benzamide) |
| *N*,*N'*-(2*S*,2'*S*)-1,1'-(butane-1,4-diylbis(azanediyl))bis(3-methyl-1-oxobutane-2,1-diyl)bis(4-(1*H*-imidazol-5-yl)benzamide) | *N*,*N'*-(2*S*,2'*S*)-1,1'-(pentane-1,5-diylbis(azanediyl))bis(3-methyl-1-oxobutane-2,1-diyl)bis(4-(1*H*-imidazol-5-yl)benzamide) |
| *N*,*N'*-(2*S*,2'*S*)-1,1'-(hexane-1,6-diylbis(azanediyl))bis(3-methyl-1-oxobutane-2,1-diyl)bis(4-(1*H*-imidazol-5-yl)benzamide) | *N*,*N'*-(2*S*,2'*S*)-1,1'-(heptane-1,7-diylbis(azanediyl))bis(3-methyl-1-oxobutane-2,1-diyl)bis(4-(1*H*-imidazol-5-yl)benzamide) |
| *N*,*N'*-(2*S*,2'*S*)-1,1-(octane-1,8-diylbis(azanediyl))bis(3-methyl-1-oxobutane-2,1-diyl)bis(4-(1*H*-imidazol-5-yl)benzamide) | *N*,*N'*-(2*S*,2'*S*)-1,1'-(nonane-1,9-diylbis(azanediyl))bis(3-methyl-1-oxobutane-2,1-diyl)bis(4-(1*H*-imidazol-5-yl)benzamide) |

(continued)

| | |
|---|---|
| *N*,*N'*-(2*S*,2'*S*)-1,1'-(decane-1,10-diylbis(azanediyl))bis(3-methyl-1-oxobutane-2,1-diyl)bis(4-(1*H*-imidazol-5-yl)benzamide) | N,N'-(2*S*,2'*S*)-1,1'-(undecane-1,11-diylbis(azanediyl))bis(3-methyl-1-oxobutane-2,1-diyl)bis(4-(1*H*-imidazol-5-yl)benzamide) |
| *N*,*N'*-(2*S*,2'*S*)-1,1'-(dodecane-1,12-diylbis(azanediyl))bis(3-methyl-1-oxobutane-2,1-diyl)bis(4-(1*H*-imidazol-5-yl)benzamide) | |
| 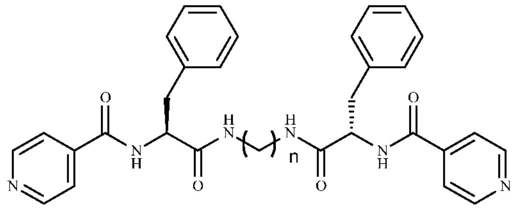 | |
| | *N*,*N*'-(2*S*,2'*S*)-1,1'-(ethane-1,2-diylbis(azanediyl))bis(1-oxo-3-phenylpropane-2,1-diyl)diisonicotinamide |
| *N*,*N*'-(2*S*,2'*S*)-1,1'-(profane-1,3-diylbis(azanediyl))bis(1-oxo-3-phenylpropane-2,1-diyl)diisonicotinamide | *N*,*N*'-(2*S*,2'*S*)-1,1'-(butane-1,4-diylbis(azanediyl))bis(1-oxo-3-phenylpropane-2,1-diyl)diisonicotinamide |
| *N*,*N*'-(2*S*,2'*S*)-1,1'-(pentane-1,5-diylbis(azanediyl))bis(1-oxo-3-phenylpropane-2,1-diyl)diisonicotinamide | *N*,*N*'-(2*S*,2'*S*)-1,1'-(hexane-1,6-diylbis(azanediyl))bis(1-oxo-3-phenylpropane-2,1-diyl)diisonicotinamide |
| *N*,*N*'-(2*S*,2'*S*)-1,1'-(heptane-1,7-diylbis(azanediyl))bis(1-oxo-3-phenylpropane-2,1-diyl)diisonicotinamide | *N*,*N*'-(2*S*,2'*S*)-1,1'-(octane-1,8-diylbis(azanediyl))bis(1-oxo-3-phenylpropane-2,1-diyl)diisonicotinamide |
| *N*,*N*'-(2*S*,2'*S*)-1,1'-(nonane-1,9-diylbis(azanediyl))bis(1-oxo-3-phenylpropane-2, 1-diyl)diisonicotinamide | *N*,*N*'-(2*S*,2'*S*)-1,1'-(decant-1,10-diylbis(azanediyl))bis(1-oxo-3-phenylpropane-2,1-diyl)diisonicotinamide |
| *N*,*N*'-(2*S*,2'*S*)-1,1'-(undecane-1,11-diylbis(azanediyl))bis(1-oxo-3-phenylpropane-2,1-diyl)diisonicotinamide | *N*,*N*'-(2*S*,2'*S*)-1,1'-(dodecane-1,12-diylbis(azanediyl))bis(1-oxo-3-phenylpropane-2,1-diyl)diisonicotinamide |
| *N*,*N*'-(2*S*,2'*S*)-1,1'-(tridecane-1,13-diylbis(azanediyl))bis(1-oxo-3-phenylpropane-2,1-diyl)diisonicotinamide | *N*,*N*'-(2*S*,2'*S*)-1,1'-(tetradecane-1,14-diylbis(azanediyl))bis(1-oxo-3-phenylpropane-2,1-diyl)diisonicotinamide |
| *N*,*N*'-(2*S*,2'*S*)-1,1'-(hexadecane-1,16-diylbis(azanediyl))bis(1-oxo-3-phenylpropane-2, 1-diyl)diisonicotinamide | *N*,*N*'-(2*S*,2'*S*)-1,1'-(octadecane-1,18-diylbis(azanediyl))bis(1-oxo-3-phenylpropane-2,1-diyl)diisonicotinamide |
| 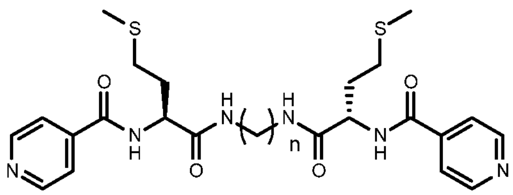 | |
| *N*-[(1*S*)-3-methylsulfanyl-1-[2-[[(2*S*)-4-methylsulfanyl-2-(pyridine-4-carbonylamino)butanoyl]amino]ethylcarbamoyl]propyl]pyridine-4-carboxamide | *N*-[(1*S*)-3-methylsulfanyl-1-[3-[[(2*S*)-4-methylsulfanyl-2-(pyridine-4-carbonylamino)butanoyl]amino]propylcarbamoyl]propyl]pyridine-4-carboxamide |

(continued)

| | |
|---|---|
| *N*-[(1*S*)-3-methylsulfanyl-1-[4-[[(2*S*)-4-methylsulfanyl-2-(pyridine-4-carbonylamino)butanoyl]amino]butylcarbamoyl]propyl]pyridine-4-carboxamide | *N*-[(1*S*)-3-methylsulfanyl-1-[5-[[(2*S*)-4-methylsulfanyl-2-(pyridine-4-carbonylamino)butanoyl]amino]pentylcarbamoyl]propyl]pyridine-4-carboxamide |
| *N*-[(1*S*)-3-methylsulfanyl-1-[6-[[(2*S*)-4-methylsulfanyl-2-(pyridine-4-carbonylamino)butanoyl]amino]hexylcarbamoyl]propyl]pyridine-4-carboxamide | *N*-[(1*S*)-3-methylsulfanyl-1-[7-[[(2*S*)-4-methylsulfanyl-2-(pyridine-4-carbonylamino)butanoyl]amino]heptylcarbamoyl]propyl]pyridine-4-carboxamide |
| *N*-[(1*S*)-3-methylsulfanyl-1-[8-[[(2*S*)-4-methylsulfanyl-2-(pyridine-4-carbonylamino)butanoyl]amino]octylcarbamoyl]propyl]pyridine-4-carboxamide | *N*-[(1*S*)-3-methylsulfanyl-1-[9-[[(2*S*)-4-methylsulfanyl-2-(pyridine-4-carbonylamino)butanoyl]amino]nonylcarbamoyl]propyl]pyridine-4-carboxamide |
| *N*-[(1*S*)-3-methylsulfanyl-1-[10-[[(2*S*)-4-methylsulfanyl-2-(pyridine-4-carbonylamino)butanoyl]amino]decylcarbamoyl]propyl]pyridine-4-carboxamide | *N*-[(1*S*)-3-methylsulfanyl-1-[11-[[(2*S*)-4-methylsulfanyl-2-(pyridine-4-carbonylamino)butanoyl]amino]undecylcarbamoyl]propyl]pyridine-4-carboxamide |
| *N*-[(1*S*)-3-methylsulfanyl-1-[12-[[(2*S*)-4-methylsulfanyl-2-(pyridine-4-carbonylamino)butanoyl]amino]docylcarbamoyl]propyl]pyridine-4-carboxamide | |

In one aspect, said pH tuneable di-amido gellants is selected from the group consisting of:

*N*,*N*'-(2*S*,2'*S*)-1,1'-(propane-1,3-diylbis(azanediyl))bis(3-methyl-1-oxobutane-2,1-diyl) diisonicotinamide,

*N*,*N*'-(2*S*,2'*S*)-1,1'-(octane-1,8-diylbis(azanediyl))bis(3-methyl-1-oxobutane-2,1-diyl) diisonicotinamide,

*N*,*N*'-(2*S*,2'*S*)-1,1'-(dodecane-1,12-diylbis(azanediyl))bis(3-methyl-1-oxobutane-2,1-diyl) diisonicotinamide,

*N*-[(1*S*)-2-methyl-1-[3-[[(2*S*)-3-methyl-2-(pyridine-4-carbonylamino)pentanoyl]amino] propylcarbamoyl]butyl]pyridine-4-carboxamide,

*N*-[(1*S*)-2-methyl-1-[8-[[(2*S*)-3-methyl-2-(pyridine-4-carbonylamino)pentanoyl]amino] octylcarbamoyl]butyl]pyridine-4-carboxamide,

*N*-[(1*S*)-2-methyl-1-[12-[[(2*S*)-3-methyl-2-(pyridine-4-carbonylamino)pentanoyl]amino] dodecylcarbamoyl]butyl]pyridine-4-carboxamide,

*N*-[(1*S*)-3-methylsulfanyl-1-[3-[[(2*S*)-4-methylsulfanyl-2-(pyridine-4-carbonylamino)butanoyl] amino]propylcarbamoyl]propyl]pyridine-4-carboxamide,

*N*-[(1*S*)-3-methylsulfanyl-1-[8-[[(2*S*)-4-methylsulfanyl-2-(pyridine-4-carbonylamino)butanoyl] amino]octylcarbamoyl]propyl]pyridine-4-carboxamide,

*N*-[(1*S*)-3-methylsulfanyl-1-[12-[[(2*S*)-4-methylsulfanyl-2-(pyridine-4-carbonylamino)butanoyl] amino]dodeccylcarbamoyl]propyl]pyridine-4-carboxamide,

(6*S*,14*S*')-6,14-diisopropyl-4,7,13,16-tetraoxo-5,8,12,15-tetraazanonadecane-1,19-dioic acid,

(6*S*,19*S*)-6,19-diisopropyl-4,7,18,21-tetraoxo-5,8,17,20-tetraazatetracosane-1,24-dioic acid,

(6*S*,23*S*)-6,23-diisopropyl-4,7,22,25-tetraoxo-5,8,21,24-tetraazaoctacosane-1,28-dioic acid, and mixtures thereof.

In certain embodiments of both types of pH tuneable di-amido gellant structures, AA comprises at least one

of: Alanine, ß-Alanine and substituted Alanines; Linear Amino-Alkyl Carboxylic Acid; Cyclic Amino-Alkyl Carboxylic Acid; Aminobenzoic Acid Derivatives; Aminobutyric Acid Derivatives; Arginine and Homologues; Asparagine; Aspartic Acid; p-Benzoyl-Phenylalanine; Biphenylalanine; Citrulline; Cyclopropylalanine; Cyclopentylalanine; Cyclohexylalanine; Cysteine, Cystine and Derivatives; Diaminobutyric Acid Derivatives; Diaminopropionic Acid; Glutamic Acid Derivatives; Glutamine; Glycine; Substituted Glycines; Histidine; Homoserine; Indole Derivatives; Isoleucine; Leucine and Derivatives; Lysine; Methionine; Naphthylalanine; Norleucine; Norvaline; Ornithine; Phenylalanine; Ring-Substituted Phenylalanines; Phenylglycine; Pipecolic Acid, Nipecotic Acid and Isonipecotic Acid; Proline; Hydroxyproline; Thiazolidine; Pyridylalanine; Serine; Statine and Analogues; Threonine; Tetrahydronorharman-3-carboxylic Acid; 1,2,3,4-Tetrahydroisoquinoline; Tryptophane; Tyrosine; Valine; and combinations thereof.

The pH tuneable di-amido gellant molecule may also comprise protective groups, preferably from 1 to 2 protective groups, preferably two protective groups. Examples of suitable protective groups are provided in "Protecting Groups", P.J. Kocienski, ISBN 313 135601 4, Georg Thieme Verlag, Stutgart; and "Protective Groups in Organic Chemistry", T.W. Greene, P.G.M. Wuts, ISBN 0-471-62301-6, John Wiley& Sons, Inc, New York.

The pH tuneable di-amido gellant preferably has a minimum gelling concentration (MGC) of from 0.1 to 100 mg/mL in the fluid detergent composition, at the target pH of the composition, preferably from 0.1 to 25 mg/mL, more preferred from 0.5 mg/mL to 10 mg/mL in accordance with the MGC Test Method. The MGC as used herein can be represented as mg/ml or as a wt %, where wt% is calculated as the MGC in mg/ml divided by 10. In one embodiment, when measured in the fluid detergent composition, the MGC is from 0.1 to 100 mg/mL, preferably from 0.1 mg/mL to 25 mg/mL of the pH tuneable amido gellant, more preferably from 0.5 mg/mL to 10 mg/mL, or at least 0.1 mg/mL, at least 0.3 mg/mL, at least 0.5 mg/mL, at least 1.0 mg/mL, at least 2.0 mg/mL, at least 5.0 mg/mL of pH tuneable amido gellant. Though in some embodiments microcapsule compositions or fluid detergent compositions may have a pH-tuneable di-amido gellant concentration either above or below the MGC, the pH tuneable amido gellants may result in particularly useful rheologies below the MGC.

*Secondary External Structurants*

In one embodiment, the pH tuneable di-amido gellant may be combined with from 0.01% to 5% by weight of one or more additional external structurants, based on the weight of the microcapsule composition. Without being limited by theory, it is believed that the use of an additional external structurant permits improved control of the time-dependent gelling. For example, while the pH tuneable di-amido gellant provides ultimately superior gelling, other external structurants may provide a temporary gel structure while the pH tuneable di-amido gellant is still undergoing gelling. Non-limiting examples of suitable secondary structurants are:

(i) *Bacterial Cellulose:* The microcapsule composition may also comprise from 0.005% to 1.0% by weight of a bacterial cellulose network. The term "bacterial cellulose" encompasses any type of cellulose produced via fermentation of a bacteria of the genus *Acetobacter* such as *CELLULON® by* CPKelco U.S. and includes materials referred to popularly as microfibrillated cellulose, reticulated bacterial cellulose, and the like.

(ii) *Coated Bacterial Cellulose:* In one embodiment, the bacterial cellulose is at least partially coated with a polymeric thickener, for instance as prepared in accordance with the methods disclosed in US 2007/0027108 paragraphs 8 to 19. In one embodiment the at least partially coated bacterial cellulose comprises from 0.1% to 5%, preferably from 0.5% to 3.0 %, by weight of bacterial cellulose; and from 10% to 90% by weight of the polymeric thickener. Suitable bacterial cellulose include the bacterial cellulose described above and suitable polymeric thickeners include: carboxymethylcellulose, cationic hydroxymethylcellulose, and mixtures thereof.

(iii) *Non-Polymeric Crystalline Hydroxyl-Functional Materials*: In a preferred embodiment, the microcapsule composition further comprises from 0.01 to 1% by weight of the composition of a non-polymeric crystalline, hydroxyl functional structurant. Such non-polymeric crystalline, hydroxyl functional structurants generally comprise a crystallizable glyceride which can be pre-emulsified to aid dispersion into the final fluid detergent composition. Preferred crystallizable glycerides include hydrogenated castor oil or "HCO" or derivatives thereof, provided that it is capable of crystallizing in the liquid detergent composition.

(iv) *Polymeric Structuring Agents*: Microcapsule compositions may comprise from 0.01% to 5% by weight of a naturally derived and/or synthetic polymeric structurant. Examples of naturally derived polymeric structurants include: hydroxyethyl cellulose, hydrophobically modified hydroxyethyl cellulose, carboxymethyl cellulose, polysaccharide derivatives and mixtures thereof. Examples of synthetic polymeric structurants include: polycarboxylates, polyacrylates, hydrophobically modified ethoxylated urethanes, hydrophobically modified non-ionic polyols and mixtures thereof. In another preferred embodiment, the polyacrylate is a copolymer of unsaturated mono- or di-carbonic acid and $C_1$-$C_{30}$ alkyl ester of the (meth)acrylic acid.

Microcapsules with Benefit Agents

In one aspect disclosed herein, the population of encapsulates includes, for example at least 80%, at least 85%, or even at least 90% of the encapsulates, comprising a shell wall and a core, the shell comprising a polymer forms a shell wall that encapsulates the core, the core comprising a benefit agent. In some applications, it may be desirable to incorporate a population of encapsulates that releases its contents upon application of a suitable mechanical force together, with a population of encapsulates that releases its content upon exposure to light.

The shell wall of the microcapsules comprise a polymer which may be selected from the group consisting of melamine-formaldehyde resins, urea-formaldehyde resins, polyamides, polyacrylates, melamine-dimethoxyethanol crosslinked with formaldehyde, polyacrylamide, silica, polystyrene cross linked with divinylbenzene, polyacrylate based materials, polyacrylate formed from metthylmethacrylate/dimethylaminomethyl methacrylate, polyacrylate formed from amine acrylate and/or methacrylate and a strong acid, polyacrylate formed from a carboxylic acid acrylate and/or methacrylate monomer and a strong base; polyacrylate formed from an amine acrylate and/or methacrylate monomer and a carboxylic acid acrylate and/or carboxylic acid methacrylate monomer, silicone, cross linked silicone, urea crosslinked with formaldehyde, urea crosslinked with gluteraldehyde, gelatin,polyacrylates, acrylate monomers, polyvinyl alcohol, polyvinyl acetate, polyurethanes, polyureas, polyesters, polycarbonates, polysaccharides derivatives, such as alginate, chitosan, methyl cellulose, hydroxypropyl methylcellulose phthalate, cellulose acetate phthalate, and mixtures thereof.

The polymer containing shell wall of the microcapsules may be surrounded and/or coated by a polymer selected from the group consisting of a cationic, non-ionic or anionic polymer, such as polyvinylformaldehyde, partially hydroxylated polyvinylformaldehyde, polyvinylamine, polyethyleneimine, ethoxylated polyethyleneimine, polyvinylalcohol, polyacrylates, and combinations thereof

The benefit agent within such encapsulates may be the same or different, depending on the application. The benefit agent may be selected from the group consisting of perfumes, silicones, drugs, sensates, mosquito repellants, vitamins, herbicide, insecticide, fertilizer, suds suppressor, dyes, hueing agents, phase transition materials, pheromones, sweeteners, hormones, attractants, skin benefit agents, lubricants, cooling agents, oils, biocontrol agents and combinations thereof

In one aspect, the benefit agent comprises a perfume composition, the perfume composition comprising perfume raw materials having a cLogP of from about 2.0 to about 4.5, or even from about 2.5 to about 4.25.

In one aspect of the encapsulate, the encapsulate's core may comprise a perfume composition selected from the group consisting of:

a) a perfume composition having a cLog P of less than 4.5 to about 2, less than 4.25 to about 2.2, less than 4.0 to about 2.5 or even less than 3.75 to about 2.6;
b) a perfume composition comprising, based on total perfume composition weight, at least 60% or even at least 70% perfume materials having a cLog P of less than 4.0 to about 2.0;
c) a perfume composition comprising, based on total perfume composition weight, at least 35%, at least 50% or even at least 60% perfume materials having a cLog P of less than 3.5 to about 2;
d) a perfume composition comprising, based on total perfume composition weight, at least 40% perfume materials having a cLog P of less than 4.0 to about 2.0 or even less than 3.5 to about 2.0 and at least 1% perfume materials having a cLog P of less than 2.0 to about 1.0;
e) a perfume composition comprising, based on total perfume composition weight, at least 40% perfume materials having a cLog P of less than 4.0 to about 2 or even less than 3.5 to about 2.0 and at least 15% perfume materials having a cLog P of less than 3.0 to about 1.5;
f) a perfume composition comprising, based on total perfume composition weight, at least 1% or even at least 2.0% of a butanoate ester and at least 1% of a pentanoate ester;
g) a perfume composition comprising, based on total perfume composition weight, at least 2.0% or even at least 3.0% of an ester comprising an allyl moiety and at least 10%, at least 25% or even at least 30% of another perfume comprising an ester moiety;
h) a perfume composition comprising, based on total perfume composition weight, at least 1.0% or even at least 5.0% of an aldehyde comprising an alkyl chain moiety;
i) a perfume composition comprising, based on total perfume composition weight, at least 2.0% of a butanoate ester;
j) a perfume composition comprising, based on total perfume composition weight, at least 1.0% of a pentanoate ester;
k) a perfume composition comprising, based on total perfume composition weight, at least 3.0% of an ester comprising an allyl moiety and at least 1.0% of an aldehyde comprising an alkyl chain moiety;
l) a perfume composition comprising, based on total perfume composition weight, at least 25% of a perfume comprising an ester moiety and at least 1.0% of an aldehyde comprising an alkyl chain moiety; and
m) a perfume composition comprising, based on total perfume composition weight, from about 0.5% to about 50%, from about 1.0% to about 40%, or even from about 5.0% to about 30% of a parametric balancing agent.

with the proviso that the perfume composition does not contain or has less than 10% based on total weight composition of perfume raw materials containing the same functional groups than the monomers used for its encapsulation.

In another aspect, the benefit agent comprises silicone, antibacterial agents, flavors, heating or cooling agents. Other suitable benefit agents include flavor ingredients including spices or flavor enhancers that contribute to the overall flavor perception of the product into which the benefit agent delivery system is incorporated. Pharmaceutical benefit agents may include drugs. In one embodiment, a therapeutically acceptable amount of drug is employed.

In another aspect, biocontrol agents including biocides, antimicrobials, bactericides, fungicides, algaecides, mildewcides, disinfectants, sanitizer-like bleaches, antiseptics, insecticides, insect and/or moth repellant, vermicides, plant growth hormones, and the like are employed. In another aspect, antimicrobials including glutaraldehyde, cinnamaldehyde, and mixtures thereof are employed. In another aspect, azole antimicrobials may be employed as the benefit agent, wherein such azole antimicrobials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof.

In another aspect, typical insect and/or moth repellants such as citronellal, citral, N, N diethyl meta toluamide, Rotundial, 8-acetoxycarvotanacenone, and mixtures thereof may be employed. Other examples of insect and/or moth repellant for use as benefit agents herein are disclosed in US 4,449,987, 4,693,890, 4,696,676, 4,933,371, 5,030,660, 5,196,200, and "Semio Activity of Flavor and Fragrance molecules on various Insect Species", B.D. Mookherjee et al., published in Bioactive Volatile Compounds from Plants, ASC Symposium Series 525, R. Teranishi, R.G. Buttery, and H. Sugisawa, 1993, pp. 35-48. These publications are incorporated herein by reference.

*Suitable Perfume Raw Materials*

When the core of the encapsulates comprise a perfume as the benefit agent, in some embodiments the perfume or perfumes in the encapsulate core may be chosen such that the 1% to 30% of the perfume raw materials have ClogP less than 3 and boiling point less than 250° C., known as quadrant 1 perfume raw materials, and more than 70% of the perfume raw materials are selected from the group consisting of those having ClogP greater than 3 or ClogP less than 3, with a boiling point of greater than 250° C., known as quadrant 2, 3 an 5 perfume raw materials. Suitable Quadrant I, II, III and IV perfume raw materials are disclosed in U.S. Pat. No. 6,869,923 B1. However, in general the perfume or perfumes may be chosen from any perfume that delivers a desired benefit to the microcapsule composition and/or fluid detergent compositions comprising the microcapsule composition.

*Parametric Balancing Agents*

The encapsulates disclosed herein optionally may further comprise a parametric balancing agent. As used herein, a "balancing agent" is a material that can be employed to alter one or more of the following properties of an encapsulate and/or the encapsulate's core material: density, vapor pressure and/or ClogP. When a balancing agent is used to alter the vapor pressure of an encapsulate and/or the encapsulate's core material, the boiling of such encapsulate and/or the encapsulate's core material is inherently altered.

In one aspect, at least a portion of the parametric balancing agent, when present, is contained in the shell wall or shell of the encapsulate. In another aspect, the core of the encapsulate may comprise at least a portion of the parametric balancing agent.

In one aspect, the parametric balancing agent may be a density balancing agent. Without being bound by theory, density balancing agents are materials that are able to balance the density of an encapsulate so that such encapsulate can be stably suspended in a fluid consumer good. In one aspect of the encapsulate, the encapsulate may have a settling velocity of less than about 1.5 cm/year, less than about 1.0 cm/year. In another aspect of the encapsulate, the perfume composition may comprise one or more fluids and may have a density such that the density ratio of the encapsulate and at least one of the one or more fluids is from about 0.9:1 to about 1.1:1. Suitable density balancing agents include: brominated vegetable oil, Tint Ayd PC 9003 and those listed in USPA 29035365 A1.

For example, the density balancing agents may be metal oxides selected from but not limited to titanium dioxide ($TiO_2$), zinc oxide (ZnO), $Fe_2O_3$, $CO_2O_3$, CoO, NiO, AgO, CuO, zicornium dioxide ($ZrO_2$), silica, and other metal oxides. They should have specific density of greater than unity. Oxides that can function both as densification agent and provide additional functional properties are particularly useful.

In one aspect, the density of the density balancing agent is greater than 1. By adding density balancing agents to the core, the density of the encapsulate can be independently adjusted to a desired level. Hydrophobically modified metal oxides are useful. Examples of metal oxides include, but are not limited to, Uvinul® TiO2, Z-COTE® HP1, T-lite™ SF. T-lite™ SF-S, T-lite™ MAX, and Z-COTE® MAX manufactured by BASF; Aerosil® R812, Aerosil® R972/R94 from

Evonik; and Ti-Pure® R-700, and Ti-Select™ TS-6200 from Dupont.

The density balancing agents may also be selected from organic compounds including brominated vegetable oil (BVO) and sucrose acetate isobutyrate. Such density balancing agents are available from Eastman chemical (Kingsport, Tenn. 37662) under the trade name: Sustane SAIB, Sustane SAIB MCT, Sustane SAIB ET-10, Eastman SAIB-100, Eastman SAIB-90EA, and Eastman SAIB-90. For the purpose of densification, any substances that possesses a density of greater than 1 and does not significantly react with the fragrance may be used. Furthermore, a material that is odorless or does not interfere with the primary odor of the fragrance is particularly useful. The selection can be made based on the chemical and physical compatibility of the densification agent and that of the fragrance core.

The density balancing agents may also be selected from inert metallic particles or metallic compounds or metallic alloys since these materials normally posses density of greater than 1.0 and can be highly effective in providing the desired density. Examples are silver (Ag), zinc (Zn), iron (Fe), cobalt (Co), Nickel (Ni), and copper (Cu). Useful materials are those compatible with the fragrance core.

In the case of a solid density balancing agent, the material can be of any physical dimension and morphology compatible with the desired encapsulate characteristics (e.g., size). The core materials can be selected from materials with dimensions ranging from a few nanometers to microns. As far as the physical dimension is concerned, the upper and lower limit of the core densification agent will be ultimately determined by the physical dimension of the encapsulates. For example, if one is to prepare a 30 micron densified capsule, the maximum physical dimension of the densification agent is limited to 30 micron or less. It is possible that, for optimal performance, there might exist a relationship between the physical dimension of the capsule and that of the core densification agent. For example, a larger capsule may need a densification agent with a larger physical size for better breakage and release. This may be explainable if the capsules breakage is by protrusion force. Likewise, a smaller capsule may benefit from material with a smaller grain size.

The core materials may further be hollow, porous, meso-porous, nano-porous or completely filled. The core materials can also be of any regular or irregular shape including sphere, square, needles, fibers, and ellipsoids. The physical dimension of the core materials can range from nanoscaled to micro-sized materials. The densification agents in the core can have any dimension, as long as they can be encapsulated in the polyamide encapsulating shell and as long as the fragrance core remains liquid after the fragrance core is mixed with the densification agent.

*cLogP balancing agents*: Without being bound by theory, cLogP balancing agents are materials able to increase the total cLogP of the benefit agent composition in order to facilitate the emulsification of the benefit agent composition.

*Vapor pressure balancing agents*: the vapor pressure provides a gauge of the rate of evaporation and the odor strength of the perfume composition. While not being bound by theory, when the vapor pressure of the encapsulate's core is balanced, the encapsulate provides a longer lasting and more consistent core material release.

Materials having a low vapor pressure (i.e., with a b.p. over 250 °C) may be used to improve the longevity of the release (see table below), or materials with a high vapor pressure (i.e., with a b.p. at or below 250 °C) may be used for a fast release.

*Adjunct ingredients of Microcapsule composition*

The non-limiting list of adjuncts illustrated hereinafter are suitable for use in the instant compositions and may be desirably incorporated in certain embodiments described herein, for example to buffer the pH, for treatment of encapsulation reaction residues, or to stabilize the microcapsule composition as is the case with scavengers, salts, structuring systems or the like. It should be understood that such adjuncts are in addition to the components that are supplied via the encapsulates. The precise nature of these additional components, and levels of incorporation thereof, will depend on the physical form of the composition and the nature of the operation for which it is to be used. Suitable adjunct materials include, but are not limited to, surfactants, processing aids and/or pigments.

i) aminoplast encapsulation processes may need a formaldehyde scavenger. Said formaldehyde scavenger may be selected from the group consisting of β-dicarbonyl compounds, amides, imines, acetal formers, sulfur containing compounds, activated carbon, ammonium, organic amines, an oxidizing agent and mixtures thereof

*Process of Making Microcapsule Composition*

A process for making a Microcapsule composition comprising encapsulates, the microcapsule composition comprising a pH tuneable di-amido gellant, may comprise:

(a) premixing the encapsulates with the vehicle;
(b) preparing a premix comprising a pH tuneable di-amido gellant, wherein the premix is at a pH such that the pH tuneable di-amidi gellant is in its ionic, non-viscosity building form;
(c) combining the encapsulate mixture with the gellant premix;

(c) adjusting the pH of the microcapsule composition as needed, such that the microcapsule composition is at a pH at which the pH tuneable di-amido gellant is in its nonionic, viscosity building, form.

The encapsulate/vehicle mixture and the gellant premix are preferably processed such that the temperatures of the gellant premix and/or the encapsulate mixture stream are maintained at less than about 50°C, preferably less than about 30°C; particularly if the microcapsule composition further incorporates encapsulates comprising thermosensitive benefit agents, such as enzymes.

Consumer Product / Fluid Detergent Compositions

The stable microcapsule compositions of the present invention may be incorporated into liquid or gel consumer product. As used herein "consumer product" means baby care, beauty care, fabric & home care, family care, feminine care, health care, snack and/or beverage products or devices intended to be used or consumed in the form in which it is sold, and not intended for subsequent commercial manufacture or modification. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

As used herein, the term "cleaning and/or treatment composition" includes, unless otherwise indicated, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, dentifrice, car or carpet shampoos, bathroom cleaners; hair shampoos and hair-rinses; shower gels and foam baths and metal cleaners; as well as cleaning auxiliaries such as bleach additives and "stain-stick" or pre-treat types, substrate-laden products such as dryer added sheets, dry and wetted wipes and pads, nonwoven substrates, and sponges; as well as sprays and mists.

As used herein, the term "fabric care composition" includes, unless otherwise indicated, fabric softening compositions, fabric enhancing compositions, fabric freshening compositions and combinations thereof.

In particular embodiments the microcapsule compositions described above may be incorporated into a fluid detergent composition.

Fluid detergent compositions as described herein include, but are not limited to, consumer products such as: shampoos; skin cleaners and exfolients; shaving liquids, foams and gels; products for treating fabrics, hard surfaces and any other surfaces in the area of fabric and home care, including: dishwashing, laundry cleaning, laundry and rinse additives, hard surface cleaning including floor and toilet bowl cleaners; products relating to oral care including toothpastes and gels and whiteners. A particularly preferred embodiment is a "fluid laundry detergent composition." As used herein, "fluid laundry detergent composition" refers to any laundry treatment composition comprising a fluid capable of wetting and cleaning fabric e.g., clothing, in a domestic washing machine.

The fluid detergent composition can include solids or gases in suitably subdivided form, but the overall composition excludes product forms which are non-fluid overall, such as tablets or granules. The fluid detergent compositions preferably have densities in the range from of 0.9 $g/cm^3$ to 1.3 $g/cm^3$, more preferably from 1.00 $g/cm^3$ to 1.10 $g/cm^3$, excluding any solid additives but including any bubbles, if present.

According to some embodiments, a fluid detergent composition may comprise a microcapsule composition according to any of the embodiments described above, and at least one of the adjunct ingredients described above such as, for example, surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments.

*Unit Dose Detergent:*

In some embodiments, the fluid detergent composition may be enclosed within a water soluble pouch material. Preferred polymers, copolymers or derivatives thereof suitable for use in pouch materials are selected from the group: polyvinyl alcohols, polyvinyl pyrrolidone, polyalkylene oxides, acrylamide, acrylic acid, cellulose, cellulose ethers, cellulose esters, cellulose amides, polyvinyl acetates, polycarboxylic acids and salts, polyaminoacids or peptides, polyamides, polyacrylamide, copolymers of maleic/acrylic acids, polysaccharides including starch and gelatin, natural gums such as xanthum and carragum. More preferred polymers are selected from polyacrylates and water-soluble acrylate copolymers, methylcellulose, carboxymethylcellulose sodium, dextrin, ethylcellulose, hydroxyethyl cellulose, hydroxypropyl methylcellulose, maltodextrin, polymethacrylates, and most preferably selected from polyvinyl alcohols, polyvinyl alcohol copolymers and hydroxypropyl methyl cellulose (HPMC), and combinations thereof.

Adjunct ingredients of Fluid Detergent Composition

The non-limiting list of adjuncts illustrated hereinafter are suitable for use in the instant compositions and may be desirably incorporated in certain embodiments described herein, for example to assist or enhance performance, for treatment of the substrate to be cleaned, or to modify the aesthetics of the composition as is the case with perfumes, colorants, dyes or the like. It should be understood that such adjuncts are in addition to the components that are supplied via the encapsulates, agglomerates and/or slurries. The precise nature of these additional components, and levels of incorporation thereof, will depend on the physical form of the composition and the nature of the operation for which it is to be used. Suitable adjunct materials include, but are not limited to, surfactants, builders, chelating agents, dye transfer inhibiting agents, dispersants, enzymes, and enzyme stabilizers, catalytic materials, bleach activators, polymeric dispersing agents, clay soil removal/anti-redeposition agents, brighteners, suds suppressors, dyes, additional perfume and perfume delivery systems, structure elasticizing agents, fabric softeners, carriers, hydrotropes, processing aids and/or pigments. In addition to the disclosure below, suitable examples of such other adjuncts and levels of use are found in U.S. Pat. Nos. 5,576,282, 6,306,812 B1 and 6,326,348 B1.

Each adjunct ingredient is not essential to the microcapsule compositions described above but may be useful or beneficial in fluid detergent compositions comprising the microcapsule composition. Thus, preferred embodiments of microcapsule compositions described herein do not contain any of the following adjuncts materials: bleach activators, surfactants, builders, chelating agents, dye transfer inhibiting agents, dispersants, enzymes, and enzyme stabilizers, catalytic metal complexes, polymeric dispersing agents, clay and soil removal/anti-redeposition agents, brighteners, suds suppressors, dyes, additional perfumes and perfume delivery systems, structure elasticizing agents, fabric softeners, carriers, hydrotropes, processing aids and/or pigments. However, when one or more adjuncts are present, such one or more adjuncts may be present as detailed below:

*Anionic and Nonionic Surfactants:*

*Anionic surfactants:* According to some embodiments, fluid detergent compositions may comprise from 1% to 70%, preferably from 5% to 60% by weight, more preferably from 10% to 50%, and most preferably from 15% to 45% by weight of a surfactant selected from the group consisting of: anionic, nonionic surfactants and mixtures thereof. The preferred ratio of anionic to nonionic surfactant is from 100:0 (i.e. no nonionic surfactant) to 5:95, more preferably from 99:1 to 1:4, most preferably 5:1 to 1.5:1.

The fluid detergent compositions may comprise from 1% to 50%, preferably from 5% to 40%, more preferably from 10% to 30% by weight of one or more anionic surfactants. Preferred anionic surfactant are selected from the group consisting of: $C_{11}$-$C_{18}$ alkyl benzene sulfonates, $C_{10}$-$C_{20}$ branched-chain and random alkyl sulfates, $C_{10}$-$C_{18}$ alkyl ethoxy sulfates, mid-chain branched alkyl sulfates, mid-chain branched alkyl alkoxy sulfates, $C_{10}$-$C_{18}$ alkyl alkoxy carboxylates comprising 1-5 ethoxy units, modified alkylbenzene sulfonate, $C_{12}$-$C_{20}$ methyl ester sulfonate, $C_{10}$-$C_{18}$ alpha-olefin sulfonate, $C_6$-$C_{20}$ sulfosuccinates, and mixtures thereof. However, by nature, every anionic surfactant known in the art of detergent compositions may be used, such as those disclosed in "Surfactant Science Series", Vol. 7, edited by W. M. Linfield, Marcel Dekker. However, the fluid detergent compositions may comprise preferably at least one sulphonic acid surfactant, such as a linear alkyl benzene sulphonic acid, or the water-soluble salt forms.

Anionic sulfonate or sulfonic acid surfactants suitable for use herein include the acid and salt forms of linear or branched $C_5$-$C_{20}$, more preferably $C_{10}$-$C_{16}$, most preferably $C_{11}$-$C_{13}$ alkylbenzene sulfonates, $C_5$-$C_{20}$ alkyl ester sulfonates, $C_6$-$C_{22}$ primary or secondary alkane sulfonates, $C_5$-$C_{20}$ sulfonated polycarboxylic acids, and mixtures thereof. The aforementioned surfactants can vary widely in their 2-phenyl isomer content.

Anionic sulphate salts suitable for use in the detergent compositions include: primary and secondary alkyl sulphates, having a linear or branched alkyl or alkenyl moiety having from 9 to 22 carbon atoms, more preferably from 12 to 18 carbon atoms; beta-branched alkyl sulphate surfactants; and mixtures thereof.

Mid-chain branched alkyl sulphates or sulfonates are also suitable anionic surfactants for use in the detergent compositions. Preferred are the $C_5$-$C_{22}$, preferably $C_{10}$-$C_{20}$ mid-chain branched alkyl primary sulphates. When mixtures are used, a suitable average total number of carbon atoms for the alkyl moieties is preferably within the range of from 14.5 to 17.5. Preferred mono-methyl-branched primary alkyl sulphates are selected from the group consisting of the 3-methyl to 13-methyl pentadecanol sulphates, the corresponding hexadecanol sulphates, and mixtures thereof. Dimethyl derivatives or other biodegradable alkyl sulphates having light branching can similarly be used.

Other suitable anionic surfactants for use herein include fatty methyl ester sulphonates and/or alkyl ethyoxy sulphates (AES) and/or alkyl polyalkoxylated carboxylates (AEC). Mixtures of anionic surfactants can be used, for example mixtures of alkylbenzenesulphonates and AES.

The anionic surfactants are typically present in the form of their salts with alkanolamines or alkali metals such as sodium and potassium. Preferably, the anionic surfactants are neutralized with alkanolamines such as monoethanolamine or triethanolamine, and are fully soluble in the liquid phase.

*Nonionic Surfactants:* The fluid detergent compositions may comprise up to 30%, preferably from 1% to 15%, more preferably from 2% to 10% by weight of one or more nonionic surfactants. Suitable nonionic surfactants include, but are not limited to $C_{12}$-$C_{18}$ alkyl ethoxylates ("AE") including the so-called narrow peaked alkyl ethoxylates, $C_6$-$C_{12}$ alkyl phenol alkoxylates (especially ethoxylates and mixed ethoxy/propoxy), block alkylene oxide condensate of $C_6$-$C_{12}$ alkyl phenols, alkylene oxide condensates of $C_8$-$C_{22}$ alkanols and ethylene oxide/propylene oxide block polymers (Pluronic®-BASF Corp.), as well as semi polar nonionics (e.g., amine oxides and phosphine oxides). An extensive disclosure of suitable nonionic surfactants can be found in U.S. Pat. 3,929,678.

Alkylpolysaccharides such as disclosed in U.S. Pat. 4,565,647 are also useful nonionic surfactants for the detergent compositions. Also suitable are alkyl polyglucoside surfactants. In some embodiments, suitable nonionic surfactants include those of the formula $R_1(OC_2H_4)_nOH$, wherein $R_1$ is a $C_{10}$-$C_{16}$ alkyl group or a $C_8$-$C_{12}$ alkyl phenyl group, and n is from 3 to about 80. In some embodiments, the nonionic surfactants may be condensation products of $C_{12}$-$C_{15}$ alcohols with from 5 to 20 moles of ethylene oxide per mole of alcohol, e.g., $C_{12}$-$C_{13}$ alcohol condensed with about 6.5 moles of ethylene oxide per mole of alcohol. Additional suitable nonionic surfactants include polyhydroxy fatty acid amides of the formula:

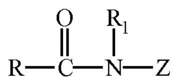

wherein R is a $C_9$-$C_{17}$ alkyl or alkenyl, $R_1$ is a methyl group and Z is glycidyl derived from a reduced sugar or alkoxylated derivative thereof. Examples are N-methyl N-1-deoxyglucityl cocoamide and N-methyl N-1-deoxyglucityl oleamide.

<u>*Additional Surfactants*</u>

The fluid detergent compositions may comprise additional surfactant selected from the group consisting: anionic, cationic, nonionic, amphoteric and/or zwitterionic surfactants and mixtures thereof.

*Cationic surfactants:* Suitable cationic surfactants can be water-soluble, water-dispersable or water-insoluble. Such cationic surfactants have at least one quaternized nitrogen and at least one long-chain hydrocarbyl group. Compounds comprising two, three or even four long-chain hydrocarbyl groups are also included. Examples include alkyltrimethylammonium salts, such as $C_{12}$ alkyltrimethylammonium chloride, or their hydroxyalkyl substituted analogs. Compositions known in the art may comprise, for example, 1% or more of cationic surfactants.

*Amphoteric and/or zwitterionic surfactants:* Suitable amphoteric or zwitterionic detersive surfactants of use in the fluid detergent compositions include those which are known for use in hair care or other personal care cleansing. Non-limiting examples of suitable zwitterionic or amphoteric surfactants are described in U.S. Pat. Nos. 5,104,646 (Bolich Jr. et al.), 5,106,609 (Bolich Jr. et al.).

Amphoteric detersive surfactants suitable for use in the composition include those surfactants broadly described as derivatives of aliphatic secondary and tertiary amines in which the aliphatic radical can be straight or branched chain and wherein one of the aliphatic substituents contains from 8 to 18 carbon atoms and one contains an anionic group such as carboxy, sulfonate, sulfate, phosphate, or phosphonate. Suitable amphoteric detersive surfactants include, but are not limited to: cocoamphoacetate, cocoamphodiacetate, lauroamphoacetate, lauroamphodiacetate, and mixtures thereof.

Zwitterionic detersive surfactants suitable for use in the compositions are well known in the art, and include those surfactants broadly described as derivatives of aliphatic quaternary ammonium, phosphonium, and sulfonium compounds, in which the aliphatic radicals can be straight or branched chain, and wherein one of the aliphatic substituents contains from 8 to 18 carbon atoms and one contains an anionic group such as carboxy, sulfonate, sulfate, phosphate or phosphonate. Zwitterionics such as betaines may be used.

Furthermore, amine oxide surfactants having the formula: $R(EO)_x(PO)_y(BO)_zN(O)(CH_2R')_2.qH_2O$ (I) are also useful in the detergent compositions. R is a relatively long-chain hydrocarbyl moiety which can be saturated or unsaturated, linear or branched, and can contain from 8 to 20, preferably from 10 to 16 carbon atoms, and is more preferably $C_{12}$-$C_{16}$ primary alkyl. R' is a short-chain moiety preferably selected from hydrogen, methyl and $-CH_2OH$. When x+y+z is different from 0, EO is ethyleneoxy, PO is propyleneneoxy and BO is butyleneoxy. Amine oxide surfactants are illustrated by $C_{12}$-$C_{14}$ alkyldimethyl amine oxide.

Non-limiting examples of other anionic, zwitterionic, amphoteric or optional additional surfactants suitable for use in the compositions are described in McCutcheon's, Emulsifiers and Detergents, 1989 Annual, published by M. C. Publishing Co., and U.S. Pat. Nos. 3,929,678, 2,658,072; 2,438,091; 2,528,378.

*Enzymes*

The fluid detergent compositions may comprise from 0.0001% to 8 % by weight of a detersive enzyme which provides cleaning performance and/or fabric care benefits. Such compositions have a neat pH of from 6 to 10.5. Suitable enzymes include proteases, amylases, cellulases, lipases, xylogucanases, pectate lyases, mannanases, bleaching enzymes, cutinases, and mixtures thereof. A preferred enzyme combination comprises a cocktail of conventional detersive enzymes such as lipase, protease, cellulase and amylase. Detersive enzymes are described in greater detail in U.S. Patent No. 6,579,839.

*Enzyme Stabilizers*

Suitable mass efficient reversible protease inhibitors for the inhibition of serine proteases would include derivates of boronic acid, especially derivatives of phenyl boronic acid and peptide aldehydes, including tripeptide aldehydes. Examples of such compounds are disclosed in WO 98/13458 A1, WO 07/113241 A1, and USP 5,972,873.

The stabilizer may be selected from the group consisting of thiophene-2 boronic acid, thiophene-3 boronic acid, acetamidophenyl boronic acid, benzofuran-2 boronic acid, naphtalene-1 boronic acid, naphtalene-2 boronic acid, 2-fomyl phenyl boronic acid (2-FPBA), 3-FBPA, 4-FPBA, 1-thianthrene boronic acid, 4-dibenzofuran boronic acid, 5-methylthiophene-2 boronic, acid, thionaphtrene boronic acid, furan-2 boronic acid, furan-3 boronic acid, 4,4 biphenyldiboronic acid, 6-hydroxy-2-naphtalene, 4-(methylthio) phenyl boronic acid, 4 (trimethylsilyl)phenyl boronic acid, 3-bromothiophene boronic acid, 4-methylthiophene boronic acid, 2-naphtyl boronic acid, 5-bromothiphene boronic acid, 5-chlorothiophene boronic acid, dimethylthiophene boronic acid, 2-bromophenyl boronic acid, 3-chlorophenyl boronic acid, 3-methoxy-2-thiophene, p-methyl-phenylethyl boronic acid, 2-thianthrene boronic acid, dibenzothiophene boronic acid, 4-carboxyphenyl boronic acid, 9-anthryl boronic acid, 3,5 dichlorophenyl boronic, acid, diphenyl boronic acidanhydride, o-chlorophenyl boronic acid, p-chlorophenyl boronic acid m-bromophenyl boronic acid, p-bromophenyl boronic acid, p-fluorophenyl boronic acid, p-tolyl boronic acid, o-tolyl boronic acid, octyl boronic acid, 1,3,5 trimethylphenyl boronic acid, 3-chloro-4-flourophenyl boronic acid, 3-aminophenyl boronic acid, 3,5-bis-(trifluoromethyl) phenyl boronic acid, 2,4 dichlorophenyl boronic acid, 4-methoxyphenyl boronic acid and mixtures thereof. Further suitable boronic acid derivatives suitable as stabilizers are described in USP 4,963,655, USP 5,159,060, WO 95/12655, WO 95/29223, WO 92/19707, WO 94/04653, WO 94/04654, USP 5,442,100, USP 5,488,157 and USP 5,472,628.

Suitable mass efficient reversible protease inhibitors may comprise 4-formyl phenyl boronic acid.

The mass efficient reversible protease inhibitor may comprise a reversible peptide protease inhibitor. Examples of suitable reversible peptide protease inhibitors and processes for making same may be found in USP 6,165,966 and WO 98/13459 A1.

Suitable tripeptide enzyme inhibitors may have the following structure:

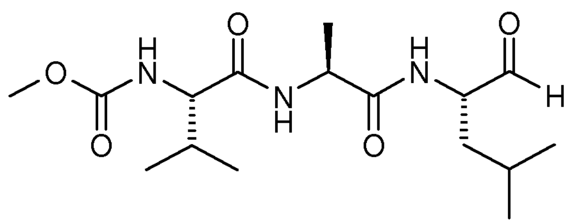

The mass efficient reversible protease inhibitor may comprise a protease inhibitor of the protein type such as RASI, BASI, WASI (bifunctional alpha-amylase/subtilisin inhibitors of rice, barley and wheat) as disclosed in WO09/095425 or SSI (streptomyces subtilisin inhibitor) and variants thereof as disclosed in Protein Engineering Design & Selection, vol 17 no.4, p.333-339, 2004.

*Polymer Deposition Aids*

Preferably, the fluid detergent composition comprises from 0.1% to 7%, more preferably from 0.2% to 3%, of a polymer deposition aid. As used herein, "polymer deposition aid" refers to any cationic polymer or combination of cationic polymers that significantly enhance deposition of a care benefit agents onto substrates (such as fabric) during washing (such as laundering). Suitable polymer deposition aids can comprise a cationic polysaccharide and/or a copolymer. "Fabric care benefit agent" as used herein refers to any material that can provide fabric care benefits. Non-limiting examples of fabric care benefits include, but are not limited to: fabric softening, color protection, color restoration, pill/fuzz reduction, anti-abrasion and anti-wrinkling. Non-limiting examples of fabric care benefit agents include: silicone derivatives, oily sugar derivatives, dispersible polyolefins, polymer latexes, cationic surfactants and combinations thereof.

*Cleaning Polymers*

The detergent compositions herein may optionally contain from 0.01 to 10% by weight of one or more cleaning polymers that provide for broad-range soil cleaning of surfaces and fabrics and/or suspension of the soils. Any suitable cleaning polymer may be of use. Useful cleaning polymers are described in US 2009/0124528A1. Non-limiting examples of useful categories of cleaning polymers include: amphiphilic alkoxylated grease cleaning polymers; clay soil cleaning polymers; soil release polyers; and soil suspending polymers.

*Bleaching systems*

One embodiment is a composition, wherein the composition is a fluid laundry bleach additive comprising from 0.1% to 12% by weight of a bleach or bleach system, preferably a peroxide bleach, and further comprises a neat pH of from 2 to 6. Another embodiment is a fluid laundry detergent composition comprising: from 0.1% to 12 % by weight of the bleach, and a composition pH of from 6.5 to 10.5. Suitable hydrogen peroxide sources are described in detail in Kirk Othmer's Encyclopedia of Chemical Technology, 4th Ed (1992, John Wiley & Sons), Vol. 4, pp. 271-300 "Bleaching Agents (Survey)", and include the various forms of sodium perborate and sodium percarbonate, including various coated and modified forms. For example, hydrogen peroxide itself; perborates, e.g., sodium perborate (any hydrate but preferably the mono- or tetra-hydrate); sodium carbonate peroxyhydrate or equivalent percarbonate salts; sodium pyrophosphate peroxyhydrate, urea peroxyhydrate, or sodium peroxide can be used herein. Also useful are sources of available oxygen such as persulfate bleach (e.g., OXONE, manufactured by DuPont). Sodium perborate monohydrate and sodium percarbonate are particularly preferred. The detergent compositions may also comprise as the bleaching agent a chlorine-type bleaching material. Such agents include for example sodium dichloroisocyanurate ("NaDCC"). However, chlorine-type bleaches are less preferred for compositions comprising enzymes. The bleaching systems may also include ingredients selected from the group consisting of: bleach activators, hydrogen peroxide, hydrogen peroxide sources, organic peroxides, metal-containing bleach catalysts, transition metal complexes of macropolycyclic rigid ligands, other bleach catalysts, preformed peracids, photobleaches and mixtures thereof.

*Bleach Activators:* The peroxygen bleach component in the composition can be formulated with an activator (peracid precursor), present at levels of from 0.01 to 15 %, preferably from 0.5 to 10 %, more preferrably from 1% to 8 % by weight of the composition. Preferred activators are selected from the group consisting of: tetraacetyl ethylene diamine (TAED), benzoylcaprolactam (BzCL), 4-nitrobenzoylcaprolactam, 3-chlorobenzoylcaprolactam, benzoyloxybenzenesulphonate (BOBS), nonanoyloxybenzenesulphonate (NOBS), phenyl benzoate (PhBz), decanoyloxybenzenesulphonate ($C_{10}$-OBS), benzoylvalerolactam (BZVL), octanoyloxybenzenesulphonate ($C_8$-OBS), perhydrolyzable esters and mixtures thereof, alternatively benzoylcaprolactam and benzoylvalerolactam, 4-[N-(nonaoyl) amino hexanoyloxy]-benzene sulfonate sodium salt (NACA-OBS) (See U.S. 5,523,434), dodecanoyloxy-benzenesulphonate (LOBS or $C_{12}$-OBS), 10-undecenoyloxybenzenesulfonate (UDOBS or $C_{11}$-OBS with unsaturation in the 10 position), and decanoyloxybenzoic acid (DOBA) and mixtures thereof. Non-limiting examples of suitable bleach activators, including quaternary substituted bleach activators, are described in US 6,855,680.

*Hydrogen Peroxides sources:* Suitable examples include inorganic perhydrate salts, including alkali metal salts such as sodium salts of perborate (usually mono- or tetra-hydrate), percarbonate, persulphate, perphosphate, persilicate salts and mixtures thereof. When employed, inorganic perhydrate salts are typically present in amounts of from 0.05% to 40%, preferably from 1% to 30% by weight of the composition.

*Organic Peroxides:* Diacyl Peroxides that do not cause visible spotting or filming are particularly preferred. One example is dibenzoyl peroxide. Other suitable examples are illustrated in Kirk Othmer, Encyclopedia of Chemical Technology at 27-90, v. 17, John Wiley and Sons, (1982).

*Metal-containing Bleach Catalysts:* Preferred bleach catalysts include manganese and cobalt-containing bleach catalysts. Other suitable metal-containing bleach catalysts include catalyst systems comprising a transition metal cation of defined bleach catalytic activity, such as copper, iron, titanium, ruthenium tungsten, molybdenum, or manganese

cations; an auxiliary metal cation having little or no bleach catalytic activity, such as zinc or aluminum cations; and a sequestrate having defined stability constants for the catalytic and auxiliary metal cations, particularly ethylenediaminetetraacetic acid, ethylenediaminetetra (methylenephosphonic acid) and water-soluble salts thereof. Suitable catalyst systems are disclosed in US 4,430,243.

*Transition Metal Complexes of Macropolycyclic Rigid Ligands:* The fluid detergent compositions herein may also include bleach catalysts comprising a transition metal complex of a macropolycyclic rigid ligand. The amount used is preferably more than 1 ppb, more preferably 0.001 ppm or more, even more preferably from 0.05 ppm to 500 ppm (wherein "ppb" denotes parts per billion by weight and "ppm" denotes parts per million by weight).

*Other Bleach Catalysts:* Other bleach catalysts such as organic bleach catalysts and cationic bleach catalysts are suitable for the fluid detergent compositions. Organic bleach catalysts are often referred to as bleach boosters. The fluid detergent compositions herein may comprise one or more organic bleach catalysts to improve low temperature bleaching. Preferred organic bleach catalysts are zwitterionic bleach catalysts, including aryliminium zwitterions. Suitable examples include 3-(3,4-dihydroisoquinolinium) propane sulfonate and 3,4-dihydro-2-[2-(sulfooxy)decyl]isoquinolimium. Suitable aryliminium zwitterions include:

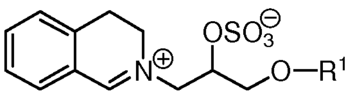

wherein $R^1$ is a branched alkyl group containing from 9 to 24 carbons or linear alkyl group containing from 11 to 24 carbons.

Preferably, each $R^1$ is a branched alkyl group containing from 9 to 18 carbons or linear alkyl group containing from 11 to 18 carbons, more preferably each $R^1$ is selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other suitable examples of organic bleach catalysts can be found in US 5,576,282 and US 5,817,614, EP 923,636 B1, WO 2001/16263 A1, WO 2000/42156 A1, WO 2007/001262 A1.

Suitable examples of cationic bleach catalysts are described in US 5,360,569, US 5,442,066, US 5,478,357, US 5,370,826, US 5,482,515, US 5,550,256, WO 95/13351, WO 95/13352, and WO 95/13353.

*Preformed peracids:* The preferred preformed peracid is Phthalimido peroxycaproic acid (PAP). Other suitable preformed peracids include, but are not limited to, compounds selected from the group consisting of: percarboxylic acids and salts, percarbonic acids and salts, perimidic acids and salts, peroxymonosulfuric acids and salts, and mixtures thereof. In compositions such as bleach containing fluid laundry detergents, the preformed peracid may be present at a level of from 0.1% to 25%, preferably from 0.5% to 20%, more preferably from 1% to 10%, most preferably from 2% to 4% by weight of the composition. Alternatively, higher levels of peracid may be present. For instance, compositions such as fluid laundry bleach additives may comprise from 10% to 40%, preferably from 15% to 30%, more preferably from 15% to 25% by weight preformed peracid.

*Optical brighteners*

These are also known as fluorescent whitenening agents for textiles. Preferred levels are from 0.001% to 1% by weight of the fluid detergent composition. Suitable brighteners are disclosed in EP 686691B and include hydrophobic as well as hydrophilic types. In some embodiments, Brightener 49 may be especially preferred.

*Hueing Dyes*

Hueing dyes or fabric shading dyes are useful adjuncts in fluid detergent compositions. Suitable dyes include blue and/or violet dyes having a hueing or shading effects. The fluid detergent compositions herein may comprise from 0.00003% to 0.1%, preferably from 0.00008% to 0.05%, more preferably from 0.0001% to 0.04% by weight of the fabric hueing dye.

*Particulate material*

The fluid detergent composition may include particulate material such as clays, suds suppressors, encapsulated sensitive ingredients, e.g., perfumes including perfume microcapsules, bleaches and enzymes in encapsulated form; or aesthetic adjuncts such as pearlescent agents including mica, pigment particles, or the like. Suitable levels are from 0.0001% to 5%, or from 0.1% to 1% by weight of the fluid detergent composition.

*Perfume and odor control agents*

In preferred embodiments, the fluid detergent composition comprises a perfume. If present, perfume is typically incorporated at a level from 0.001 to 10%, preferably from 0.01% to 5%, more preferably from 0.1% to 3% by weight of the composition. The perfume may comprise a perfume microcapsule and/or a perfume nanocapsule. In other embodiments, the fluid detergent composition comprises odour control agents such as uncomplexed cyclodextrin as described in US 5,942,217.

*Hydrotropes*

The fluid detergent composition optionally comprises a hydrotrope in an effective amount, i.e. up to 15%, preferably 1% to 10%, more preferably 3% o 6% by weight, so that the fluid detergent compositions are compatible in water. Suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in US 3,915,903.

TEST METHODS

*Minimum gelling concentration (MGC)*

MGC is calculated by a tube inversion method based on R.G. Weiss, P. Terech; "Molecular Gels: Materials with self-assembled fibrillar structures" 2006 springer, p 243. In order to determine the MGC, three screenings are done:

(a) First screening: prepare several vials increasing the pH tuneable di-amido gellant concentration from 0.5 % to 5.0% by weight in 0.5% steps, at the target pH.
(b) Determine in which interval the gel is formed (one inverted sample still flowing and the next one is already a strong gel). In case no gel is formed at 5%, higher concentrations are used.
(c) Second screening: prepare several vials increasing the pH tuneable di-amido gellant concentration in 0.1 weight % steps in the interval determined in the first screening, at the target pH.
(d) Determine in which interval the gel is formed (one inverted sample still flowing and the next one is already a strong gel)
(e) Third screening: in order to have a very precise percentage of the MGC, run a third screening in 0.025 weight % steps in the interval determined in the second screening, at the target pH.
(f) The Minimum Gelling Concentration (MGC) is the lowest concentration which forms a gel in the third screening (does not flow on inversion of the sample).

For each screening, samples are prepared and treated as follows: 8mL vials (Borosilacate glass with Teflon cap, ref. B7857D, Fisher Scientific Bioblock) are filled with 2.0000±0.0005 g (KERN ALJ 120-4 analytical balance with ± 0.1mg precision) of the vehicle for which we want to determine the MGC. The vial is sealed with the screw cap and left for 10 minutes in an ultrasound bath (Elma Transsonic T 710 DH, 40 kHz, 9.5L, at 25 °C and operating at 100% power) in order to disperse the solid in the liquid. Complete dissolution is then achieved by heating, using a heating gun (Bosch PHG-2), and gentle mechanical stirring of the vials. It is crucial to observe a completely clear solution. Handle vials with care. While they are manufactured to resist high temperatures, a high solvent pressure may cause the vials to explode. Vials are cooled to 25°C, for 10min in a thermostatic bath (Compatible Control Thermostats with controller CC2, D77656, Huber). Vials are inverted, left inverted for 1 minute, and then observed for which samples do not flow. After the third screening, the concentration of the sample that does not flow after this time is the MGC. For those skilled in the art, it is obvious that during heating solvent vapours may be formed, and upon cooling down the samples, these vapours can condense on top of the gel. When the vial is inverted, this condensed vapour will flow. This is discounted during the observation period. If no gels are obtained in the concentration interval, higher concentrations must be evaluated.

*pH measurement of a liquid detergent composition*

pH measurement of a microcapsule composition or liquid detergent composition may be measured using test method EN 1262.

EXAMPLES

100 grams of following microcapsule compositions are produced and stored in a sealed 100 mL graduated recipient at 40°C over 2 weeks to reproduce transport conditions over sea.

| Ingredient | REFERENCE Percentage (%)* | A | B | C |
|---|---|---|---|---|
| Aminoplast Perfume Encapsulates (containing 87% of a perfume) | 32.5 | 32.5 | 32.5 | 32.5 |
| Acetoacetamide | 0.03 | 0.03 | 0.03 | 0.03 |
| Xanthan gum (Kelzan ASX-T from CP Kelco) | - | 0.3 | - | - |
| Hydrogenated castor oil[1] | - | - | 0.3 | - |
| *N*,*N*'-(2*S*,2'*S*)-1,1'-(dodecane-1,12-diylbis(azanediyl))bis(3-methyl-1-oxobutane-2,1-diyl)diisonicotinamide[2] | - | - | - | 0.1 |
| *Water* | Up to 100% | Up to 100% | Up to 100% | Up to 100% |

*percentage in weight based on total microcapsule composition weight
[1]added as a premix containing 4% hydrogenated castor oil
[2]added as a premix containing 10% *N*,*N*'-(2*S*,2'*S*)-1,1'-(dodecane-1,12-diylbis(azanediyl))bis(3-methyl-1-oxobutane-2,1-diyl)diisonicotinamide

After this time, the amount of phase split is measured and represented as percentage of phase split using following formula:

$$\%\ phase\ split = \frac{mL\ lower\ phase}{total\ mL} \# 100$$

wherein the total mL are 100 mL (according to the 100 mL graduated recipient used) and the mL lower phase, are the amount of mL from the microcapsule composition after the phase split (usually encapsulates cream on the top, leaving a watery phase below)

| | REFERENCE | A | B | C |
|---|---|---|---|---|
| % Phase Split | 30.8 | 14.8 | 18.5 | 0 |

Thus, it is clear that robust, stable microcapsule compositions can be formed and transport during long periods at high temperatures, when a pH-tuneable di-amido gellant is included in the microcapsule composition.

All percentages, ratios and proportions used herein are by weight percent of the composition, unless otherwise specified. All average values are calculated "by weight" of the composition or components thereof, unless otherwise expressly indicated.

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

Though particular embodiments have been illustrated and described, it should be obvious to those skilled in the art that various other changes and modifications can be made without departing from the scope of the invention. It is therefore intended to cover in the appended claims all such changes and modifications that are within the scope of this invention.

## Claims

1. A microcapsule composition comprising:

a) a vehicle, preferably water;
b) a population of encapsulates, each encapsulate comprising:

i) a core comprising at least one benefit agent; and
ii) a shell wall surrounding the core, the shell wall comprising at least one polymer;

c) a pH-tuneable di-amido gellant having formula [I]

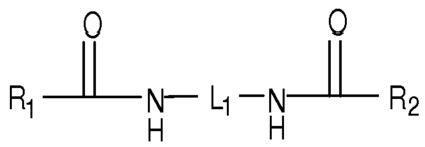

where $R_1$ and $R_2$ are aminofunctional end-groups; $L_1$ is a backbone moiety having molecular weight from 14 g/mol to 500 g/mol; and at least one of $L_1$, $R_1$ or $R_2$ comprises a pH-sensitive group, wherein the pH-tuneable di-amino gellant has a $pK_a$ of from 1 to 30, preferably from 1.5 to 14.

**2.** The microcapsule composition of claim 1, wherein the pH tuneable di-amido gellant has a molecular weight from 150 g/mol to 1500 g/mol.

**3.** The microcapsule composition of claim 1, wherein the pH tuneable di-amido gellant has a minimum gelling concentration (MGC) of from 0.1 mg/mL to 100 mg/mL at the pH of the microcapsule composition.

**4.** The microcapsule composition of claim 1, wherein the pH tuneable di-amido gellant is selected from the group: *N*,*N*'-(2*S*,2'*S*)-1,1'-(ethane-1,2-diylhis(azanediyl))bis(3-methyl-1-oxobutane-2,1-diyl)diisonicotinamide; *N*,*N*'-(2*S*,2'*S*)-1,1'-(propane-1,3-diylbis(azanediyl))bis(3-methyl-1-oxobutane-2,1-diyl)diisonicotinamide; *N*,*N*'-(2*S*,2'*S*)-1,1'-(butane-1,4-diylbis(azanediyl))bis(3-methyl-1-oxobutane-2,1-diyl)diisonicotinamide; *N*,*N*'-(2*S*,2'*S*)-1,1'-(pentane-1,5-diylbis(azanediyl))bis(3-methyl-1-oxobutane-2,1-diyl)diisonicotinamide; *N*,*N*'-(2*S*,2'*S*)-1,1'-(hexane-1,6-diylbis(azanediyl))bis(3-methyl-1-oxobutane-2,1-diyl)diisonicotinamide; *N*,*N*'-(2*S*,2'*S*)-1,1'-(heptane-1,7-diylbis(azanediyl))bis(3-methyl-1-oxobutane-2,1-diyl)diisonicotinamide; *N*,*N*'-(2*S*,2'*S*)-1,1'-(octane-1,8-diylbis(azanediyl))bis(3-methyl-1-oxobutane-2,1-diyl)diisonicotinamide; (6*S*,13*S*')-6,13-diisopropyl-4,7,12,15-tetraoxo-5,8,11,14-tetraazaoctadecane-1,18-dioic acid; (6*S*,14*S*')-6,14-diisopropyl-4,7,13,16-tetraoxo-5,8,12,15-tetraazanonadecane-1,19-dioic acid; (6*S*,15*S*')-6,15-diisopropyl-4,7,14,17-tetraoxo-5,8,13,16-tetraazaeicosane-1,20-dioic acid; (6*S*,16*S*')-6,16-diisopropyl-4,7,15,18-tetraoxo-5,8,14,17-tetraazaheneicosane-1,21-dioic acid; (6*S*,17*S*')-6,17-diisopropyl-4,7,16,19-tetraoxo-5,8,15,18-tetraazadocosane-1,22-dioic acid; (6S,18S')-6,18-diisopropyl-4,7,17,20-tetraoxo-5,8,16,19-tetraazatricosane-1,23-dioic acid; (6*S*,19*S*')-6,19-diisopropyl-4,7,18,21-tetraoxo-5,8,17,20-tetraazatetracosane-1,24-dioic acid; (6*S*,20*S*')-6,20-diisopropyl-4,7,19,22-tetraoxo-5,8,18,21-tetraazapentacosane-1,25-dioic acid; (6S,21S')-6,21-diisopropyl-4,7,20,23-tetraoxo-5,8,19,22-tetraazahexacosane-1,26-dioic acid; (6S,22*S*')-6,22-diisopropyl-4,7,21,24-tetraoxo-5,8,20,23-tetraazaheptacosane-1,27-dioic acid; (6*S*,23*S*')-6,23-diisopropyl-4,7,22,25-tetraoxo-5,8,21,24-tetraazaoctacosane-1,28-dioic acid; *N*,*N*'-(2*S*,2'*S*)-1,1'-(ethane-1,2-diylbis(azanediyl))bis(3-methyl-1-oxobutane-2,1-diyl)bis(4-(1H-imidazol-5-yl)benzamide); *N*,*N*'-(2*S*,2'*S*)-1,1'-(propane-1,3-diylbis(azanediyl))bis(3-methyl-1-oxobutane-2,1-diyl)bis(4-(1H-imidazol-5-yl)benzamide *N*,*N*'-(2*S*,2'*S*)-1,1'-(butane-1,4-diylbis(azanediyl))bis(3-methyl-1-oxobutane-2,1-diyl)bis(4-(1H-imidazol-5-yl)benzamide); *N*,*N*'-(2*S*,2'*S*)-1,1'-(pentane-1,5-diylbis(azanediyl))bis(3-methyl-1-oxobutane-2,1-diyl)bis(4-(1H-imidazol-5-yl)benzamide); *N*,*N*'-(2*S*,2'*S*)-1,1'-(hexane-1,6-diylbis(azanediyl))bis(3-methyl-1-oxobutane-2,1-diyl)bis(4-(1H-imidazol-5-yl)benzamide); *N*,*N*'-(2*S*,2'*S*)-1,1'-(heptane-1,7-diylbis(azanediyl))bis(3-methyl-1-oxobutane-2,1-diyl)bis(4-(1H-imidazol-5-yl)benzamide); *N*,*N*'-(2*S*,2'*S*)-1,1'-(octane-1,8-diylbis(azanediyl))bis(3-methyl-1-oxobutane-2,1-diyl)bis(4-(1H-imidazol-5-yl)benzamide); *N*,*N*'-(2*S*,2'*S*)-1,1'-(nonane-1,9-diylbis(azanediyl))bis(3-methyl-1-oxobutane-2,1-diyl)bis(4-(1H-imidazol-5-yl)benzamide); *N*,*N*'-(2*S*,2'*S*)-1,1'-(decane-1,10-diylbis(azanediyl))bis(3-methyl-1-oxobutane-2,1-diyl)bis(4-(1H-imidazol-5-yl)benzamide); *N*,*N*'-(2*S*,2'*S*)-1,1'-(undecane-1,11-diylbis(azanediyl))bis(3-methyl-1-oxobutane-2,1-diyl)bis(4-(1H-imidazol-5-yl)benzamide); *N*,*N*'-(2*S*,2'*S*)-1,1'-(dodecane-1,12-diylbis(azanediyl))bis(3-methyl-1-oxobutane-2,1-diyl)bis(4-(1H-imidazol-5-yl)benzamide); *N*,*N*'-(2*S*,2'*S*)-1,1'-(nonane-1,9-diylbis(azanediyl))bis(3-methyl-1-oxobutane-2,1-diyl)diisonicotinamide; *N*,*N*'-(2*S*,2'*S*)-1,1'-(decane-1,10-diylbis(azanediyl))bis(3-methyl-1-oxobutane-2,1-diyl)diisonicotinamide; *N*,*N*'-(2*S*,2'*S*)-1,1'-(undecane-1,11-diylbis(azanediyl))bis(3-methyl-1-oxobutane-2,1-diyl)diisonicotinamide; *N*,*N*'-(2*S*,2'*S*)-1,1'-(dodecane-1,12-diylbis(azanediyl))bis(3-methyl-1-oxobutane-2,1-diyl)diisonicotinamide; *N*,*N*'-(2*S*,2'*S*)-1,1'-(tridecane-1,13-diylbis(azanediyl))bis(3-methyl-1-oxobutane-2,1-diyl)diisonicotinamide;

*N*,*N*'-(2*S*,2'*S*)-1,1'-(tetradecane-1,14-diylbis(azanediyl))bis(3-methyl-1-oxobutane-2,1-diyl)diisonicotinamide; *N*,*N*'-(2*S*,2'*S*)-1,1'-(hexadecane-1,16-diylbis(azanediyl))bis(3-methyl-1-oxobutane-2,1-diyl)diisonicotinamide; *N*,*N*'-(2*S*,2'*S*)-1,1'-(octadecane-1,18-diylbis(azanediyl))bis(3-methyl-1-oxobutane-2,1-diyl)diisonicotinamide; *N*,*N*'-(2*S*,2'*S*)-1,1'-(ethane-1,2-diylbis(azanediyl))bis(1-oxopropane-2,1-diyl)diisonicotinamide; *N*,*N*'-(2*S*,2'*S*)-1,1'-(propane-1,3-diylbis(azanediyl))bis(1-oxopropane-2,1-diyl)diisonicotinamide; *N*,*N*'-(2*S*,2'*S*)-1,1'-(butane-1,4-diylbis(azanediyl))bis(1-oxopropane-2,1-diyl)diisonicotinamide; *N*,*N*'-(2*S*,2'*S*)-1,1-(pentane-1,5-diylbis(azanediyl))bis(1-oxopropane-2,1-diyl)diisonicotinamide; *N*,*N*'-(2*S*,2'*S*)-1,1'-(hexane-1,6-diylbis(azanediyl))bis(1-oxopropane-2,1-diyl)diisonicotinamide; *N*,*N*'-(2*S*,2'*S*)-1,1'-(heptane-1,7-diylbis(azanediyl))bis(1-oxopropane-2,1-diyl)diisonicotinamide; *N*,*N*'-(2*S*,2'*S*)-1,1'-(octane-1,8-diylbis(azanediyl))bis(1-oxopropane-2,1-diyl)diisonicotinamide; *N*,*N*'-(2*S*,2'*S*)-1,1'-(nonane-1,9-diylbis(azanediyl))bis(1-oxopropane-2,1-diyl)diisonicotinamide; *N*,*N*'-(2*S*,2'*S*)-1,1'-(decane-1,10-diylbis(azanediyl))bis(1-oxopropane-2,1-diyl)diisonicotinamide; *N*,*N*'-(2*S*,2'*S*)-1,1'-(undecane-1,11-diylbis(azanediyl))bis(1-oxopropane-2,1-diyl)diisonicotinamide; *N*,*N*'-(2*S*,2'*S*)-1,1'-(dodecane-1,12-diylbis(azanediyl))bis(1-oxopropane-2,1-diyl)diisonicotinamide; *N*,*N*'-(2*S*,2'*S*)-1,1'-(tridecane-1,13-diylbis(azanediyl))bis(1-oxopropane-2,1-diyl)diisonicotinamide; *N*,*N*'-(2*S*,2'*S*)-1,1'-(tetradecane-1,14-diylbis(azanediyl))bis(1-oxopropane-2,1-diyl)diisonicotinamide; *N*,*N*'-(2*S*,2'*S*)-1,1'-(hexadecane-1,16-diylbis(azanediyl))bis(1-oxopropane-2,1-diyl)diisonicotinamide; *N*,*N*'-(2*S*,2'*S*)-1,1'-(octadecane-1,18-diylbis(azanediyl))bis(1-oxopropane-2,1-diyl)diisonicotinamide; *N*,*N*'-(2*S*,2'*S*)-1,1'-(ethane-1,2-diylbis(azanediyl))bis(1-oxo-3-phenylpropane-2,1-diyl)diisonicotinamide; *N*,*N*'-(2*S*,2'*S*)-1,1'-(propane-1,3-diylbis(azanediyl))bis(1-oxo-3-phenylpropane-2,1-diyl)diisonicotinamide; *N*,*N*'-(2*S*,2'*S*)-1,1'-(butane-1,4-diylbis(azanediyl))bis(1-oxo-3-phenylpropane-2,1-diyl)diisonicotinamide; *N*,*N*'-(2*S*,2'*S*)-1,1'-(pentane-1,5-diylbis(azanediyl))bis(1-oxo-3-phenylpropane-2,1-diyl)diisonicotinamide; *N*,*N*'-(2*S*,2'*S*)-1,1'-(hexane-1,6-diylbis(azanediyl))bis(1-oxo-3-phenylpropane-2,1-diyl)diisonicotinamide *N*,*N*'-(2*S*,2'*S*)-1,1'-(heptane-1,7-diylbis(azanediyl))bis(1-oxo-3-phenylpropane-2,1-diyl)diisonicotinamide; *N*,*N*'-(2*S*,2'*S*)-1,1'-(octane-1,8-diylbis(azanediyl))bis(1-oxo-3-phenylpropane-2,1-diyl)diisonicotinamide; *N*,*N*'-(2*S*,2'*S*)-1,1'-(nonane-1,9-diylbis(azanediyl))bis(1-oxo-3-phenylpropane-2,1-diyl)diisonicotinamide; *N*,*N*'-(2*S*,2'*S*)-1,1'-(decane-1,10-diylbis(azanediyl))bis(1-oxo-3-phenylpropane-2,1-diyl)diisonicotinamide; *N*,*N*'-(2*S*,2'*S*)-1,1'-(undecane-1,11-diylbis(azanediyl))bis(1-oxo-3-phenylpropane-2,1-diyl)diisonicotinamide; *N*,*N*'-(2*S*,2'*S*)-1,1'-(dodecane-1,12-diylbis(azanediyl))bis(1-oxo-3-phenylpropane-2,1-diyl)diisonicotinamide; *N*,*N*'-(2*S*,2'*S*)-1,1'-(tridecane-1,13-diylbis(azanediyl))bis(1-oxo-3-phenylpropane-2,1-diyl)diisonicotinamide; *N*,*N*'-(2*S*,2'*S*)-1,1'-(tetradecane-1,14-diylbis(azanediyl))bis(1-oxo-3-phenylpropane-2,1-diyl)diisonicotinamide; *N*,*N*'-(2*S*,2'*S*)-1,1'-(hexadecane-1,16-diylbis(azanediyl))bis(1-oxo-3-phenylpropane-2,1-diyl)diisonicotinamide; *N*,*N*'-(2*S*,2'*S*)-1,1'-(octadecane-1,18-diylbis(azanediyl))bis(1-oxo-3-phenylpropane-2,1-diyl)diisonicotinamide; (2S)-2-[[2-(dodecanoylamino)acetyl]amino]propanoic acid; (2S)-2-[[2-[[2-(dodecanoylamino)acetyl]amino]acetyl]amino]propanoic acid; (2*S*)-2-[[2-(dodecanoylamino)acetyl]amino]-2-phenyl-acetic acid; (2*S*)-2-[[2-(dodecanoylamino)acetyl]amino]-3-methyl-butanoic acid; 2-[[2-(dodecanoylamino)acetyl]amino]acetic acid; (2*S*)-2-[[2-(hexadecanoylamino)acetyl]amino]propanoic acid; and mixtures thereof.

**5.** The microcapsule composition of claim 4, wherein individual encapsulates in the population of encapsulates comprise from 0.001% to 10% by weight of the pH-tuneable di-amido gellant and have a shell-to-core mass ratio less than 15%.

**6.** The microcapsule composition of claim 1, wherein the at least one benefit agent is selected from the group consisting of perfumes, silicones, drugs, sensates, mosquito repellants, vitamins, herbicide, insecticide, fertilizer, suds suppressor, dyes, hueing agents, phase transition materials, pheromones, sweeteners, hormones, attractants, skin benefit agents, lubricants, cooling agents, oils, biocontrol agents and combinations thereof preferably wherein the at least one benefit agent is a perfume.

**7.** The microcapsule composition of claim 1, wherein the polymer of the shell wall of each encapsulate is selected from the group consisting of melamine-formaldehyde resins, urea-formaldehyde resins, polyamides, polyacrylates, melamine-dimethoxyethanol crosslinked with formaldehyde, polyacrylamide, silica, polystyrene cross linked with divinylbenzene, polyacrylate based materials, polyacrylate formed from methylmethacrylate/dimethylaminomethyl methacrylate, polyacrylate formed from amine acrylate and/or methacrylate and a strong acid, polyacrylate formed from a carboxylic acid acrylate and/or methacrylate monomer and a strong base; polyacrylate formed from an amine acrylate and/or methacrylate monomer and a carboxylic acid acrylate and/or carboxylic acid methacrylate monomer, silicone, cross linked silicone, urea crosslinked with formaldehyde, urea crosslinked with gluteraldehyde, gelatin,polyacrylates, acrylate monomers, polyvinyl alcohol, polyvinyl acetate, polyurethanes, polyureas, polyesters, polycarbonates, polysaccharides derivatives, such as methyl cellulose, hydroxypropyl methylcellulose phthalate, cellulose acetate phthalate, and mixtures thereof, preferably wherein the protective polymer is selected from the group consisting of melamine-formaldehyde resins, urea-formaldehyde resins, polyamides, polyacrylates, and mixtures thereof.

8. The microcapsule composition of claim 1, wherein the particles are surrounded or coated by a polymer selected from the group consisting of a cationic, non-ionic or anionic polymer, such as polyvinylformaldehyde, partially hydroxylated polyvinylformaldehyde, polyvinylamine, polyethyleneimine, ethoxylated polyethyleneimine, polyvinylalcohol, polyacrylates, and combinations thereof.

**Patentansprüche**

1. Mikrokapselzusammensetzung, umfassend:

   a) eine Trägersubstanz, vorzugsweise Wasser;
   b) eine Population von Einkapselungen, wobei jede Einkapselung umfasst:

      i) einen Kern, umfassend mindestens einen Wirkstoff; und
      ii) eine Schalenwand, die den Kern umgibt, wobei die Schalenwand mindestens eine Polymer umfasst;

   c) ein pH-einstellendes Diamido-Gelierungsmittel mit der Formel [I]

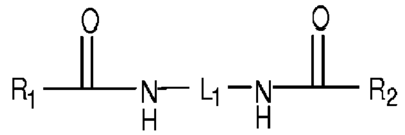

   wobei $R_1$ und $R_2$ aminofunktionelle Endgruppen sind; $L_1$ eine Grundgerüsteinheit mit einem Molekulargewicht von 14 g/mol bis 500 g/mol ist; und mindestens eines von $L_1$, $R_1$ oder $R_2$ eine pH-empfindliche Gruppe umfasst, wobei das pH-einstellende Diamido-Gelierungsmittel einen $pK_a$ von 1 bis 30, vorzugsweise von 1,5 bis 14 aufweist.

2. Mikrokapselzusammensetzung nach Anspruch 1, wobei das pH-einstellende Diamido-Gelierungsmittel ein Molekulargewicht von 150 g/mol bis 1500 g/mol aufweist.

3. Mikrokapselzusammensetzung nach Anspruch 1, wobei das pH-einstellende Diamido-Gelierungsmittel eine minimale Gelierungskonzentration (minimum gelling concentration, MGC) von 0,1 mg/mL bis 100 mg/mL bei dem pH der Mikrokapselzusammensetzung aufweist.

4. Mikrokapselzusammensetzung nach Anspruch 1, wobei das pH-einstellende Diamido-Gelierungsmittel ausgewählt ist aus der Gruppe: *N*,*N*'-(2*S*,2'*S*)-1,1'-(Ethan-1,2-diylbis(azandiyl))bis(3-methyl-1-oxobutan-2,1-diyl)diisonicotinamid; *N*,*N*'-(2*S*,2'*S*)-1,1'-(Propan-1,3-diylbis(azandiyl))bis(3-methyl-1-oxobutan-2,1-diyl)diisonicotinamid; *N*,*N*'-(2*S*,2'*S*)-1,1'-(Butan-1,4-diylbis(azandiyl))bis(3-methyl-1-oxobutan-2,1-diyl)diisonicotinamid; *N*,*N*'-(2*S*,2'*S*)-1,1'-(Pentan-1,5-diylbis(azandiyl))bis(3-methyl-1-oxobutan-2,1-diyl)diisonicotinamid; *N*,*N*'-(2*S*,2'*S*)-1,1'-(Hexan-1,6-diylbis(azandiyl))bis(3-methyl-1-oxobutan-2,1-diyl)diisonicotinamid; *N*,*N*'-(2*S*,2'*S*)-1,1'-Heptan-1,7-diylbis(azandiyl))bis(3-methyl-1-oxobutan-2,1-diyl)diisonicotinamid; *N*,*N*'-(2*S*,2'*S*)-1,1'-(Octan-1,8-diylbis(azandiyl))bis(3-methyl-1-oxobutan-2,1-diyl)diisonicotinamid; (6*S*,13*S*')-6,13-Diisopropyl-4,7,12,15-tetraoxo-5,8,11,14-tetraazaoctadecan-1,18-dicarbonsäure; (6*S*,14*S*')-6,14-Diisopropyl-4,7,13,16-tetraoxo-5,8,12,15-tetraazanonadecan-1,19-dicarbonsäure; (6*S*,15*S*')-6,15-Diisopropyl-4,7,14,17-tetraoxo-5,8,13,16-tetraazaeicosan-1,20-dicarbonsäure; (6*S*,16*S*')-6,16-Diisopropyl-4,7,15,18-tetraoxo-5,8,14,17-tetraazaheneicosan-1,21-dicarbonsäure; (6*S*,17*S*')-6,17-Diisopropyl-4,7,16,19-tetraoxo-5,8,15,18-tetraazadocosan-1,22-dicarbonsäure; (6*S*,18*S*')-6,18-Diisopropyl-4,7,17,20-tetraoxo-5,8,16,19-tetraazatricosan-1,23-dicarbonsäure; (6*S*,19*S*')-6,19-Diisopropyl-4,7,18,21-tetraoxo-5,8,17,20-tetraazatetracosan-1,24-dicarbonsäure; (6*S*,20*S*')-6,20-Diisopropyl-4,7,19,22-tetraoxo-5,8,18,21-tetraazapentacosan-1,25-dicarbonsäure; (6*S*,21*S*')-6,21-Diisopropyl-4,7,20,23-tetraoxo-5,8,19,22-tetraazahexacosan-1,26-dicarbonsäure; (6*S*,22*S*')-6,22-Diisopropyl-4,7,21,24-tetraoxo-5,8,20,23-tetraazaheptacosan-1,27-dicarbonsäure; (6*S*,23*S*')-6,23-Diisopropyl-4,7,22,25-tetraoxo-5,8,21,24-tetraazaoctacosan-1,28-dicarbonsäure; *N*,*N*'-(2*S*,2'*S*)-1,1'-(Ethan-1,2-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Propan-1,3-diylbis(azandiyl))bis(3-methyl-1-oxobutan-2,1-diyl)bis(4-(1H-imidazol-5-yl)benzamid *N*,*N*'-(2*S*,2'*S*)-1,1'-(Butan-1,4-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Pentan-1,5-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Hexan-1,6-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Heptan-1,7-diylbis(azandiyl))bis(3-methyl-1-oxobutan-2,1-diyl)bis(4-(1 H-imidazol-5-yl)benzamid); *N*,*N*'-(2*S*,2'*S*)-1,1'-(Octan-1,8-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Nonan-1,9-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Decan-1,10-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Undecan-1,11-diylbis(azandiyl))bis(3-methyl-1-oxobutan-2,1-diyl)bis(4-(1 H-imidazol-5-yl)benzamid); *N*,*N*'-(2*S*,2'*S*)-1,1'-(Dodecan-1,12-diylbis(azandiyl))bis(3-methyl-1-oxobutan-2,1-diyl)bis(4-(1H-imidazol-5-yl)benzamid); *N*,*N*'-(2*S*,2'*S*)-1,1'-(Nonan-1,9-diylbis(azandiyl))bis(3-methyl-1-oxobutan-2,1-diyl)diisonicotinamid; *N*,*N*'-(2*S*,2'*S*)-1,1'-(Decan-1,10-diylbis(azandiyl))bis(3-methyl-1-oxobutan-2,1-diyl)diisonicotinamid; *N*,*N*'-(2*S*,2'*S*)-1,1'-(Undecan-1,11-diylbis(azandiyl))bis(3-methyl-1-oxobutan-2,1-diyl)diisonicotinamid; *N*,*N*'-(2*S*,2'*S*)-1,1'-(Dodecan-1,12-diylbis(azandiyl))bis(3-methyl-1-oxobutan-2,1-diyl)diisonicotinamid; *N*,*N*'-(2*S*,2'*S*)-1,1'-(Tridecan-1,13-diylbis(azandiyl))bis(3-methyl-1-oxobutan-2,1-diyl)diisonicotinamid; *N*,*N*'-(2*S*,2'*S*)-1,1'-(Tetradecan-1,14-diylbis(azandiyl))bis(3-methyl-1-oxobutan-2,1-diyl)diisonicotinamid; *N*,*N*'-(2*S*,2'*S*)-1,1'-(Hexadecan-1,16-diylbis(azandiyl))bis(3-methyl-1-oxobutan-2,1-diyl)diisonicotinamid; *N*,*N*'-(2*S*,2'*S*)-1,1'-(Octadecan-1,18-diylbis(azandiyl))bis(3-methyl-1-oxobutan-2,1-diyl)diisonicotinamid; *N*,*N*'-(2*S*,2'*S*)-1,1'-(Ethan-1,2-diylbis(azandiyl))bis(1-oxopropan-2,1-diyl)diisonicotinamid; *N*,*N*'-(2*S*,2'*S*)-1,1'-(Propan-1,3-diylbis(azandiyl))bis(1-oxopropan-2,1-diyl)diisonicotinamid; *N*,*N*'-(2*S*,2'*S*)-1,1'-(Butan-1,4-diylbis(azandiyl))bis(1-oxopropan-2,1-diyl)diisonicotinamid; *N*,*N*'-(2*S*,2'*S*)-1,1'-(Pentan-1,5-diylbis(azandiyl))bis(1-oxopropan-2,1-diyl)diisonicotinamid; *N*,*N*'-(2*S*,2'*S*)-1,1'-(Hexan-1,6-diylbis(azandiyl))bis(1-oxopropan-2,1-diyl)diisonicotinamid; *N*,*N*'-(2*S*,2'*S*)-1,1'-(Heptan-1,7-diylbis(azandiyl))bis(1-oxopropan-2,1-diyl)diisonicotinamid; *N*,*N*'-(2*S*,2'*S*)-1,1'-(Octan-1,8-diylbis(azandiyl))bis(1-oxopropan-2,1-diyl)diisonicotinamid; *N*,*N*'-(2*S*,2'*S*)-1,1'-(Nonan-1,9-diylbis(azandiyl))bis(1-oxopropan-2,1-diyl)diisonicotinamid; *N*,*N*'-(2*S*,2'*S*)-1,1'-(Decan-1,10-diylbis(azandiyl))bis(1-oxopropan-2,1-diyl)diisonicotinamid; *N*,*N*'-(2*S*,2'*S*)-1,1'-(Undecan-1,11-diylbis(azandiyl))bis(1-oxopropan-2,1-diyl)diisonicotinamid; *N*,*N*'-(2*S*,2'*S*)-1,1'-(Dodecan-1,12-diylbis(azandiyl))bis(1-oxopropan-2,1-diyl)diisonicotinamid; *N*,*N*'-(2*S*,2'*S*)-1,1'-(Tridecan-1,13-diylbis(azandiyl))bis(1-oxopropan-2,1-diyl)diisonicotinamid; *N*,*N*'-(2*S*,2'*S*)-1,1'-(Tetradecan-1,14-diylbis(azandiyl))bis(1-oxopropan-2,1-diyl)diisonicotinamid; *N*,*N*'-(2*S*,2'*S*)-1,1'-(Hexadecan-1,16-diylbis(azandiyl))bis(1-oxopropan-2,1-diyl)diisonicotinamid; *N*,*N*'-(2*S*,2'*S*)-1,1'-(Octadecan-1,18-diylbis(azandiyl))bis(1-oxopropan-2,1-diyl)diisonicotinamid; *N*,*N*'-(2*S*,2'*S*)-1,1'-(Ethan-1,2-diylbis(azandiyl))bis(1-oxo-3-phenylpropan-2,1-diyl)diisonicotinamid; *N*,*N*'-(2*S*,2'*S*)-1,1'-(Propan-1,3-diylbis(azandiyl))bis(1-oxo-3-phenylpropan-2,1-diyl)diisonicotinamid; *N*,*N*'-(2*S*,2'*S*)-1,1'-(Butan-1,4-diylbis(azandiyl))bis(1-oxo-3-phenylpropan-2,1-diyl)diisonicotinamid; *N*,*N*'-(2*S*,2'*S*)-1,1'-(Pentan-1,5-diylbis(azandiyl))bis(1-oxo-3-phenylpropan-2,1-diyl)diisonicotinamid; *N*,*N*'-(2*S*,2'*S*)-1,1'-(Hexan-1,6-diylbis(azandiyl))bis(1-oxo-3-phenylpropan-2,1-diyl)diisonicotinamid *N*,*N*'-(2*S*,2'*S*)-1,1'-(Heptan-1,7-diylbis(azandiyl))bis(1-oxo-3-phenylpropan-2,1-diyl)diisonicotinamid; *N*,*N*'-(2*S*,2'*S*)-1,1'-(Octan-1,8-diylbis(azandiyl))bis(1-oxo-3-phenylpropan-2,1-diyl)diisonicotinamid; *N*,*N*'-(2*S*,2'*S*)-1,1'-(Nonan-1,9-diylbis(azandiyl))bis(1-oxo-3-phenylpropan-2,1-diyl)diisonicotinamid; *N*,*N*'-(2*S*,2'*S*)-1,1'-(Decan-1,10-diylbis(azandiyl))bis(1-oxo-3-phenylpropan-2,1-diyl)diisonicotinamid; *N*,*N*'-(2*S*,2'*S*)-1,1'-(Undecan-1,11-diylbis(azandiyl))bis(1-oxo-3-phenylpropan-2,1-diyl)diisonicotinamid; *N*,*N*'-(2*S*,2'*S*)-1,1'-(Dodecan-1,12-diylbis(azandiyl))bis(1-oxo-3-phenylpropan-2,1-diyl)diisonicotinamid; *N*,*N*'-(2*S*,2'*S*)-1,1'-(Tridecan-1,13-diylbis(azandiyl))bis(1-oxo-3-phenylpropan-2,1-diyl)diisonicotinamid; *N*,*N*'-(2*S*,2'*S*)-1,1'-(Tetradecan-1,14-diylbis(azandiyl))bis(1-oxo-3-phenylpropan-2,1-diyl)diisonicotinamid; *N*,*N*'-(2*S*,2'*S*)-1,1'-(Hexadecan-1,16-diylbis(azandiyl))bis(1-oxo-3-phenylpropan-2,1-diyl)diisonicotinamid; *N*,*N*'-(2*S*,2'*S*)-1,1'-(Octadecan-1,18-diylbis(azandiyl))bis(1-oxo-3-phenylpropan-2,1-diyl)diisonicotinamid; (2*S*)-2-[[2-(Dodecanoylamino)acetyl]amino]propansäure; (2*S*)-2-[[2-[[2-(Dodecanoylamino)acetyl]amino]acetyl]amino]propansäure; (2*S*)-2-[[2-(Dodecanoylamino)acetyl]amino]-2-phenyl-essigsäure; (2S)-2-[[2-(Dodecanoylamino)acetyl]amino]-3-methyl-butansäure; 2-[[2-(Dodecanoylamino)acetyl]amino]essigsäure; (2*S*)-2-[[2-(Hexadecanoylamino)acetyl]amino]propansäure; und Mischungen davon.

5. Mikrokapselzusammensetzung nach Anspruch 4, wobei einzelne Einkapselungen in der Population von Einkapselungen von 0,001 % bis 10 Gew.% des pH-einstellenden Diamido-Gelierungsmittels umfassen und ein Schale-zu-Kern-Masseverhältnis von weniger als 15 % aufweisen.

6. Mikrokapselzusammensetzung nach Anspruch 1, wobei der mindestens eine Wirkstoff ausgewählt ist aus der Gruppe bestehend aus Duftstoffen, Siliconen, Arzneistoffen, sinnlich Wahrgenommenem, Mückenabwehrmitten, Vitaminen, Herbiziden, Insektiziden, Düngemitteln, Schaumunterdrückern, Farbstoffen, Abtönmitteln, Phaseübergangsmaterialien, Pheromonen, Süßstoffen, Hormonen, Lockstoffen, hautpflegenden Wirkstoffen, Gleitmitteln, Kühlmitteln, Ölen, biohemmenden Mitteln und Kombinationen davon, vorzugsweise wobei das mindestens ein Wirkstoff ein Duftstoff ist.

7. Mikrokapselzusammensetzung nach Anspruch 1, wobei das Polymer der Schalenwand von jeder Einkapselung ausgewählt ist aus der Gruppe bestehend aus Melaminformaldehydharzen, Harnstoff-Formaldehydharzen, Polyamiden, Polyacrylaten, Melamin-Dimethoxyethanol vernetzt mit Formaldehyd, Polyacrylamid, Silica, Polystyrol vernetzt mit Divinylbenzol, Polyacrylat-basierte Materialien, Polyacrylat gebildet aus Methylmethacrylat/Dimethylaminomethylmethacrylat, Polyacrylat gebildet aus Aminacrylat und/oder Methacrylat und einer starken Säure, Polyacrylat gebildet aus einem Carboxylsäureacrylat- und/oder Methacrylat-Monomer und einer starken Base; Polyacrylat gebildet aus einem Aminacrylat- und/oder Methacrylat-Monomer und einem Carboxylsäureacrylat und/oder Carboxylsäuremethacrylat-Monomer, Silikon, vernetztem Silikon, Harnstoff vernetzt mit Formaldehyd, Harnstoff vernetzt mit Glutaraldehyd, Gelatin, Polyacrylaten, Acrylat-Monomeren, Polyvinylalkohol, Polyvinylacetat, Polyurethanen, Polyharnstoffen, Polyester, Polycarbonaten, Polysaccharid-Derivaten, wie Methylcellulose, Hydroxypropylmethylcellulosephthalat, Celluloseacetophthalat und Mischungen davon, vorzugsweise wobei das Schutzpolymer ausgewählt ist aus der Gruppe bestehend aus Melamin-Formaldehydharzen, Harnstoff-Formaldehydharzen, Polyamiden, Polyacrylaten, und Mischungen davon.

**8.** Mikrokapselzusammensetzung nach Anspruch 1, wobei die Teilchen von einem Polymer überzogen oder damit beschichtet sind, das aus der Gruppe ausgewählt ist bestehend aus kationischem, nichtionischem oder anionischem Polymer, wie Polyvinylformaldehyd, teilweise hydroxyliertes Polyvinylformaldehyd, Polyvinylamin, Polyethylenimin, ethoxyliertes Polyethylenimin, Polyvinylalkohol, Polyacrylate, und Kombinationen davon.

## Revendications

**1.** Composition de microgélule comprenant :

a) un véhicule, de préférence de l'eau ;
b) une population de gélules, chaque gélule comprenant :

i) un noyau comprenant au moins un agent bénéfique ; et
ii) une paroi de coque entourant le noyau, la paroi de coque comprenant au moins un polymère ;

c) un gélifiant di-amido à pH réglable ayant la formule [I]

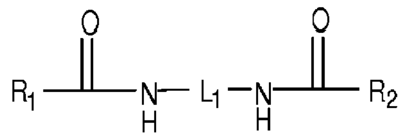

où $R_1$ et $R_2$ sont des groupes terminaux à fonction amine ; $L_1$ est un fragment de squelette ayant une masse moléculaire allant de 14 g/mol à 500 g/mol ; et au moins l'un de $L_1$, $R_1$ ou $R_2$ comprend un groupe sensible au pH, dans laquelle le gélifiant di-amido à pH réglable a un $pK_a$ allant de 1 à 30, de préférence de 1,5 à 14.

**2.** Composition de microgélule selon la revendication 1, dans laquelle le gélifiant di-amido à pH réglable a une masse moléculaire allant de 150 g/mol à 1500 g/mol.

**3.** Composition de microgélule selon la revendication 1, dans laquelle le gélifiant di-amido à pH réglable a une concentration minimale de gélification (CMG) allant de 0,1 mg/ml à 100 mg/ml au pH de la composition de microgélule.

**4.** Composition de microgélule selon la revendication 1, dans laquelle le gélifiant di-amido à pH réglable est choisi dans le groupe : *N*,*N*'-(2*S*,2'*S*)-1,1'-(éthane-1,2-diylbis(azanediyl))bis(3-méthyl-1-oxobutane-2,1-diyl)diisonicotinamide ; *N*,*N*'-(2*S*,2'*S*)-1,1'-(propane-1,3-diylbis(azanediyl))bis(3-méthyl-1-oxobutane-2,1-diyl)diisonicotinamide ; *N*,*N*'-(2*S*,2'*S*)-1,1'-(butane-1,4-diylbis(azanediyl))bis(3-méthyl-1-oxobutane-2,1-diyl)diisonicotinamide ; *N*,*N*'-(2*S*,2'*S*)-1,1'-(pentane-1,5-diylbis(azanediyl))bis(3-méthyl-1-oxobutane-2,1-diyl)diisonicotinamide ; *N*,*N*'-(2*S*,2'*S*)-1,1'-(hexane-1,6-diylbis(azanediyl))bis(3-méthyl-1-oxobutane-2,1-diyl)diisonicotinamide ; *N*,*N*'-(2*S*,2'*S*)-1,1'-(heptane-1,7-diylbis(azanediyl))bis(3-méthyl-1-oxobutane-2,1-diyl)diisonicotinamide ; *N*,*N*'-(2*S*,2'*S*)-1,1'-(octane-1,8-diylbis(azanediyl))bis(3-méthyl-1-oxobutane-2,1-diyl)diisonicotinamide ; acide (6*S*,13*S*')-6,13-diisopropyl-4,7,12,15-tétraoxo-5,8,11,14-tétra-azaoctadécane-1,18-dioïque ; acide (6*S*,14*S*')-6,14-diisopropyl-4,7,13,16-tétraoxo-5,8,12,15-tétra-azanonadécane-1,19-dioïque ; acide (6*S*,15*S*')-6,15-diisopropyl-4,7,14,17-tétraoxo-5,8,13,16-tétra-azaéicosane-1,20-dioïque ; acide (6*S*,16*S*')-6,16-diisopropyl-4,7,15,18-tétraoxo-5,8,14,17-tétra-azahénéicosane-1,21-dioïque ; acide (6*S*,17*S*')-6,17-diisopropyl-4,7,16,19-tétraoxo-5,8,15,18-tétra-azadocosane-1,22-dioïque ; acide (6*S*,18*S*')-6,18-diisopropyl-4,7,17,20-tétraoxo-5,8,16,19-tétra-azatricosane-1,23-dioïque ; acide (6*S*,19*S*')-6,19-diisopropyl-4,7,18,21-tétraoxo-5,8,17,20-tétra-azatétracosane-1,24-dioïque ; acide (6*S*,20*S*')-6,20-diisopropyl-4,7,19,22-tétraoxo-5,8,18,21-tétra-azapentacosane-1,25-dioïque ; acide (6*S*,21*S*')-6,21-diisopropyl-4,7,20,23-tétraoxo-5,8,19,22-tétra-azahexacosane-1,26-dioïque ; acide (6*S*,22*S*')-6,22-diisopropyl-4,7,21,24-tétraoxo-5,8,20,23-tétra-azaheptacosane-1,27-dioïque; acide (6*S*,23*S*')-6,23-diisopropyl-4,7,22,25-tétraoxo-5,8,21,24-tétra-azaoctacosane-1,28-dioïque ; *N*,*N*'-(2*S*,2'*S*)-1,1'-(éthane-1,2-diylbis(azanediyl))bis(3-méthyl-1-oxobutane-2,1-diyl)bis(4-(1H-imidazol-5-yl)benzamide) ; *N*,*N*'-(2*S*,2'*S*)-1,1'-(propane-1,3-diylbis(azanediyl))bis(3-méthyl-1-oxobutane-2,1-diyl)bis(4-(1H-imidazol-5-yl)benzamide *N*,*N*'-(2*S*,2'*S*)-1,1'-(butane-1,4-diylbis(azanediyl))bis(3-méthyl-1-oxobutane-2,1-diyl)bis(4-(1H-imidazol-5-yl)benzamide) ; *N*,*N*'-(2*S*,2'*S*)--1,1'-(pentane-1,5-diylbis(azanediyl))bis(3-méthyl-1-oxobutane-2,1-diyl)bis(4-(1H-imidazol-5-yl)benzamide) ; *N*,*N*'-(2*S*,2'*S*)-1,1'-(hexane-1,6-diylbis(azanediyl))bis(3-méthyl-1-oxobutane-2,1-diyl)bis(4-(1H-imidazol-5-yl)benzamide) ; *N*,*N*'-(2*S*,2'*S*)--1,1'-(heptane-1,7-diylbis(azanediyl))bis(3-méthyl-1-oxobutane-2,1-diyl)bis(4-(1H-imidazol-5-yl)benzamide) ; *N*,*N*'-(2*S*,2'*S*)-1,1'-(octane-1,8-diylbis(azanediyl))bis(3-méthyl-1-oxobutane-2,1-diyl)bis(4-(1H-imidazol-5-yl)benzamide) ; *N*,*N*'-(2*S*,2'*S*)-1,1'-(nonane-1,9-diylbis(azanediyl))bis(3-méthyl-1-oxobutane-2,1-diyl)bis(4-(1H-imidazol-5-yl)benzamide) ; *N*,*N*'-(2*S*,2'*S*)-1,1'-(décane-1,10-diylbis(azanediyl))bis(3-méthyl-1-oxobutane-2,1-diyl)bis(4-(1H-imidazol-5-yl)benzamide) ; *N*,*N*'-(2*S*,2'*S*)-1,1'-(undécane-1,11-diylbis(azanediyl))bis(3-méthyl-1-oxobutane-2,1-diyl)bis(4-(1H-imidazol-5-yl)benzamide) ; *N*,*N*'-(2*S*,2'*S*)-1,1'-(dodécane-1,12-diylbis(azanediyl))bis(3-méthyl-1-oxobutane-2,1-diyl)bis(4-(1H-imidazol-5-yl)benzamide) ; N,N'-(2S,2'S)-1,1'-(nonane-1,9-diylbis(azanediyl))bis(3-méthyl-1-oxobutane-2,1-diyl)diisonicotinamide ; *N*,*N*'-(2*S*,2'*S*)-1,1'-(décane-1,10-diylbis(azanediyl))bis(3-méthyl-1-oxobutane-2,1-diyl)diisonicotinamide ; *N*,*N*'-(2*S*,2'*S*)-1,1'-(undécane-1,11-diylbis(azanediyl))bis(3-méthyl-1-oxobutane-2,1-diyl)diisonicotinamide ; *N*,*N*'-(2*S*,2'*S*)-1,1'-(dodécane-1,12-diylbis(azanediyl))bis(3-méthyl-1-oxobutane-2,1-diyl)diisonicotinamide ; *N*,*N*'-(2*S*,2'*S*)-1,1'-(tridécane-1,13-diylbis(azanediyl))bis(3-méthyl-1-oxobutane-2,1-diyl)diisonicotinamide ; *N*,*N*'-(2*S*,2'*S*)-1,1'-(tétradécane-1,14-diylbis(azanediyl))bis(3-méthyl-1-oxobutane-2,1-diyl)diisonicotinamide ; *N*,*N*'-(2*S*,2'*S*)-1,1'-(hexadécane-1,16-diylbis(azanediyl))bis(3-méthyl-1-oxobutane-2,1-diyl)diisonicotinamide ; *N*,*N*'-(2*S*,2'*S*)-1,1'-(octadécane-1,18-diylbis(azanediyl))bis(3-méthyl-1-oxobutane-2,1-diyl)diisonicotinamide ; *N*,*N*'-(2*S*,2'*S*)-1,1'-(éthane-1,2-diylbis(azanediyl))bis(1-oxopropane-2,1-diyl)diisonicotinamide ; *N*,*N*'-(2*S*,2'*S*)-1,1'-(propane-1,3-diylbis(azanediyl))bis(1-oxopropane-2,1-diyl)diisonicotinamide ; *N*,*N*'-(2*S*,2'*S*)-1,1'-(butane-1,4-diylbis(azanediyl))bis(1-oxopropane-2,1-diyl)diisonicotinamide ; *N*,*N*'-(2*S*,2'*S*)-1,1'-(pentane-1,5-diylbis(azanediyl))bis(1-oxopropane-2,1-diyl)diisonicotinamide ; *N*,*N*'-(2*S*,2'*S*)-1,1'-(hexane-1,6-diylbis(azanediyl))bis(1-oxopropane-2,1-diyl)diisonicotinamide ; *N*,*N*'-(2*S*,2'*S*)-1,1'-(heptane-1,7-diylbis(azanediyl))bis(1-oxopropane-2,1-diyl)diisonicotinamide ; *N*,*N*'-(2*S*,2'*S*)-1,1'-(octane-1,8-diylbis(azanediyl))bis(1-oxopropane-2,1-diyl)diisonicotinamide ; *N*,*N*'-(2*S*,2'*S*)-1,1'-(nonane-1,9-diylbis(azanediyl))bis(1-oxopropane-2,1-diyl)diisonicotinamide ; *N*,*N*'-(2*S*,2'*S*)-1,1'-(décane-1,10-diylbis(azanediyl))bis(1-oxopropane-2,1-diyl)diisonicotinamide ; *N*,*N*'-(2*S*,2'*S*)-1,1'-(undécane-1,11-diylbis(azanediyl))bis(1-oxopropane-2,1-diyl)diisonicotinamide ; *N*,*N*'-(2*S*,2'*S*)-1,1'-(dodécane-1,12-diylbis(azanediyl))bis(1-oxopropane-2,1-diyl)diisonicotinamide ; *N*,*N*'-(2*S*,2'*S*)-1,1'-(tridécane-1,13-diylbis(azanediyl))bis(1-oxopropane-2,1-diyl)diisonicotinamide ; *N*,*N*'-(2*S*,2'*S*)-1,1'-(tétradécane-1,14-diylbis(azanediyl))bis(1-oxopropane-2,1-diyl)diisonicotinamide ; *N*,*N*'-(2*S*,2'*S*)-1,1'-(hexadécane-1,16-diylbis(azanediyl))bis(1-oxopropane-2,1-diyl)diisonicotinamide ; *N*,*N*'-(2*S*,2'*S*)-1,1'-(octadécane-1,18-diylbis(azanediyl))bis(1-oxopropane-2,1-diyl)diisonicotinamide ; *N*,*N*'-(2*S*,2'*S*)-1,1'-(éthane-1,2-diylbis(azanediyl))bis(1-oxo-3-phénylpropane-2,1-diyl)diisonicotinamide ; *N*,*N*'-(2*S*,2'*S*)-1,1'-(propane-1,3-diylbis(azanediyl))bis(1-oxo-3-phénylpropane-2,1-diyl)diisonicotinamide ; *N*,*N*'-(2*S*,2'*S*)-1,1'-(butane-1,4-diylbis(azanediyl))bis(1-oxo-3-phénylpropane-2,1-diyl)diisonicotinamide ; *N*,*N*'-(2*S*,2'*S*)-1,1'-(pentane-1,5-diylbis(azanediyl))bis(1-oxo-3-phénylpropane-2,1-diyl)diisonicotinamide ; *N*,*N*'-(2*S*,2'*S*)-1,1'-(hexane-1,6-diylbis(azanediyl))bis(1-oxo-3-phénylpropane-2,1-diyl)diisonicotinamide ; *N*,*N*'-(2*S*,2'*S*)-1,1'-(heptane-1,7-diylbis(azanediyl))bis(1-oxo-3-phénylpropane-2,1-diyl)diisonicotinamide ; *N*,*N*'-(2*S*,2'*S*)-1,1'-(octane-1,8-diylbis(azanediyl))bis(1-oxo-3-phénylpropane-2,1-diyl)diisonicotinamide ; *N*,*N*'-(2*S*,2'*S*)-1,1'-(nonane-1,9-diylbis(azanediyl))bis(1-oxo-3-phénylpropane-2,1-diyl)diisonicotinamide ; *N*,*N*'-(2*S*,2'*S*)-1,1'-(décane-1,10-diylbis(azanediyl))bis(1-oxo-3-phénylpropane-2,1-diyl)diisonicotinamide ; *N*,*N*'-(2*S*,2'*S*)-1,1'-(undécane-1,11-diylbis(azanediyl))bis(1-oxo-3-phénylpropane-2,1-diyl)di-isonicotinamide ; *N*,*N*'-(2*S*,2'*S*)-1,1'-(dodécane-1,12-diylbis(azanediyl))bis(1-oxo-3-phénylpropane-2,1-diyl)diisonicotinamide ; *N*,*N*'-(2*S*,2'*S*)-1,1'-(tridécane-1,13-diylbis(azanediyl))bis(1-oxo-3-phénylpropane-2,1-diyl)diisonicotinamide ; *N*,*N*'-(2*S*,2'*S*)-1,1'-(tétradecane-1,14-diylbis(azanediyl))bis(1-oxo-3-phénylpropane-2,1-diyl)di-isonicotinamide ; *N*,*N*'-(2*S*,2'*S*)-1,1'-(hexadécane-1,16-diylbis(azanediyl))bis(1-oxo-3-phénylpropane-2,1-diyl)diisonicotinamide ; *N*,*N*'-(2*S*,2'*S*)-1,1'-(octadécane-1,18-diylbis(azanediyl))bis(1-oxo-3-phénylpropane-2,1-diyl)diisonicotinamide ; acide (2*S*)-2-[[2-(dodécanoylamino)acétyl]amino]propanoïque ; acide (2*S*)-2-[[2-[[2-(dodécanoylamino)acétyl]amino]acétyl]amino]propanoïque ; acide (2*S*)-2-[[2-(dodécanoylamino)acétyl]amino]-2-phényl-acétique ; acide (2*S*)-2-[[2-(dodécanoylamino)acétyl]amino]-3-méthyl-butanoïque ; acide 2-[[2-(dodécanoylamino)acétyl]amino]acétique ; acide (2*S*)-2-[[2-(hexadécanoylamino)acétyl]amino]propanoïque ; et des mélanges de ceux-ci.

**5.** Composition de microgélule selon la revendication 4, dans laquelle des gélules individuelles dans la population de gélules comprennent de 0,001 % à 10 % en poids du gélifiant di-amido à pH réglable et ont un rapport massique de coque par rapport à noyau inférieur à 15 %.

**6.** Composition de microgélule selon la revendication 1, dans laquelle ledit au moins un agent bénéfique est choisi dans le groupe constitué de parfums, silicones, médicaments, sensates, agents répulsifs pour moustiques, vitamines, herbicide, insecticide, engrais, suppresseur de mousse, teintures, agents teintants, matériaux à transition de phase, phéromones, édulcorants, hormones, agents attirants, agents de soin cutané, lubrifiants, agents refroidissants, huiles, agents de biocontrôle et des combinaisons de ceux-ci de préférence dans laquelle ledit au moins un agent bénéfique est un parfum.

**7.** Composition de microgélule selon la revendication 1, dans laquelle le polymère de la paroi de coque de chaque gélule est choisi dans le groupe constitué de résines de mélamine-formaldéhyde, résines d'urée-formaldéhyde, polyamides, polyacrylates, mélamine-diméthoxyéthanol réticulé avec du formaldéhyde, polyacrylamide, silice, polystyrène réticulé avec du divinylbenzène, matériaux à base de polyacrylate, polyacrylate formé à partir de méthacrylate de méthyle/méthacrylate de diméthylaminométhyle, polyacrylate formé à partir d'acrylate et/ou méthacrylate d'amine et d'un acide fort, polyacrylate formé à partir d'un monomère acrylate et/ou méthacrylate d'acide carboxylique et d'une base forte ; polyacrylate formé à partir d'un monomère acrylate et/ou méthacrylate d'amine et d'un monomère acrylate d'acide carboxylique et/ou méthacrylate d'acide carboxylique, silicone, silicone réticulée, urée réticulée avec du formaldéhyde, urée réticulée avec du glutaraldéhyde, gélatine, polyacrylates, monomères acrylate, alcool polyvinylique, acétate de polyvinyle, polyuréthanes, polyurées, polyesters, polycarbonates, dérivés de polysaccharide, tels que méthylcellulose, phtalate d'hydroxypropylméthylcellulose, acétate-phtalate de cellulose, et leurs mélanges, de préférence dans laquelle le polymère de protection est choisi dans le groupe constitué de résines de mélamine-formaldéhyde, résines d'urée-formaldéhyde, polyamides, polyacrylates et leurs mélanges.

**8.** Composition de microgélule selon la revendication 1, dans laquelle les particules sont entourées ou revêtues par un polymère choisi dans le groupe constitué d'un polymère cationique, non ionique ou anionique, tel que polyvinylformaldéhyde, polyvinylformaldéhyde partiellement hydroxylé, polyvinylamine, polyéthylène-imine, polyéthylène-imine éthoxylée, alcool polyvinylique, polyacrylates, et des combinaisons de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100150975 A
- US 20060040844 A
- US 20070027108 A
- US 4449987 A
- US 4693890 A
- US 4696676 A
- US 4933371 A
- US 5030660 A
- US 5196200 A
- US 6869923 B1
- US 29035365 A1
- US 5576282 A
- US 6306812 B1
- US 6326348 B1
- US 3929678 A
- US 4565647 A
- US 5104646 A, Bolich Jr.
- US 5106609 A, Bolich Jr.
- US 2658072 A
- US 2438091 A
- US 2528378 A
- US 6579839 B
- WO 9813458 A1
- WO 07113241 A1
- US 5972873 A
- US 4963655 A
- US 5159060 A
- WO 9512655 A
- WO 9529223 A
- WO 9219707 A
- WO 9404653 A
- WO 9404654 A
- US 5442100 A
- US 5488157 A
- US 5472628 A
- US 6165966 A
- WO 9813459 A1
- WO 09095425 A
- US 20090124528 A1
- US 5523434 A
- US 6855680 B
- US 4430243 A
- US 5817614 A
- EP 923636 B1
- WO 200116263 A1
- WO 200042156 A1
- WO 2007001262 A1
- US 5360569 A
- US 5442066 A
- US 5478357 A
- US 5370826 A
- US 5482515 A
- US 5550256 A
- WO 9513351 A
- WO 9513352 A
- WO 9513353 A
- EP 686691 B
- US 5942217 A
- US 3915903 A


### Non-patent literature cited in the description


- *Chemical Reviews,* 2004, vol. 104 (3), 1201-1217
- **P.J. KOCIENSKI.** Protecting Groups. Georg Thieme Verlag
- **T.W. GREENE ; P.G.M. WUTS.** Protective Groups in Organic Chemistry. John Wiley& Sons, Inc
- **B.D. MOOKHERJEE et al.** Semio Activity of Flavor and Fragrance molecules on various Insect Species. *Bioactive Volatile Compounds from Plants*
- **R. TERANISHI ; R.G. BUTTERY ; H. SUGISAWA.** *ASC Symposium Series,* 1993, vol. 525, 35-48
- Surfactant Science Series. Marcel Dekker, vol. 7
- McCutcheon's, Emulsifiers and Detergents. M. C. Publishing Co, 1989
- *Protein Engineering Design & Selection,* 2004, vol. 17 (4), 333-339
- Bleaching Agents (Survey). Kirk Othmer's Encyclopedia of Chemical Technology. John Wiley & Sons, 1992, vol. 4, 271-300
- **KIRK OTHMER.** Encyclopedia of Chemical Technology. John Wiley and Sons, 1982, vol. 17, 27-90
- **R.G. WEISS ; P. TERECH.** Molecular Gels: Materials with self-assembled fibrillar structures. springer, 2006, 243